# EUROPÄISCHE PATENTANMELDUNG

(11) **EP 4 623 962 A1**
(43) Veröffentlichungstag der Anmeldung: **01.10.2025**
(21) Anmeldenummer: 24167557.8
(22) Anmeldetag: 28.03.2024
(51) Int. Cl.: A61M 5/315

(54) **INJEKTIONSVORRICHTUNG ZUR VERABREICHUNG EINES FLUIDEN PRODUKTS UND VERFAHREN ZUM ZUSAMMENBAUEN DIESER VORRICHTUNG**

(71) Anmelder: Ypsomed AG, 3401 Burgdorf (CH)
(72) Erfinder: Bosshard, Simon Martin, 3324 Hindelbank (CH); Hirschel, Jürg, 3007 Bern (CH); Klötzli, Urs, 3414 Oberburg (CH); Hostettler, Patrick, 3415 Hasle (CH); Tschirren, Markus, 3400 Burgdorf (CH); Schrul, Christian, 3414 Oberburg (CH)
(74) Vertreter: Eugster, Monika Katharina

(57) **Zusammenfassung**

Die Erfindung betrifft eine Injektionsvorrichtung zur Verabreichung eines fluiden Produkts in den Körper eines Patienten und ein Verfahren zum Zusammenbauen dieser Injektionsvorrichtung. Diese Injektionsvorrichtung umfasst eine mindestens zweiteilige Kolbenstange (2a; 2a'; 2a"; 2b; 2b'; 2b"), wobei der axiale Abstand zwischen dieser Kolbenstange (2a; 2a'; 2a"; 2b; 2b'; 2b") und einem in einem Behälter (3; 3') vorgesehenen Stopfen (3a; 3a') während dem Zusammenbauen der Injektionsvorrichtung bestimmt wird und derart bestimmt wird, dass ein distales Ende dieser Kolbenstange (2a; 2a'; 2a"; 2b; 2b'; 2b") und der Stopfen (3a; 3a') aneinander liegen.

## Beschreibung

Die Erfindung betrifft eine Injektionsvorrichtung zur Verabreichung eines fluiden Produkts, insbesondere eines flüssigen Medikaments von einer Injektionsvorrichtung in den Körper eines Patienten und ein Verfahren zum Zusammenbauen dieser Injektionsvorrichtung.

Der Begriff "Medikament" umfasst hier jede fliessfähige medizinische Formulierung, welche geeignet ist zur kontrollierten Verabreichung durch ein Mittel, wie z. B. eine Injektionsnadel, eine Kanüle oder eine Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vakzine, DNS oder RNS oder Oligonukleotide, Antikörper oder Teile von Antikörpern sowie geeignete Basis-, Hilfs- und Trägerstoffe.

Aus dem Stand der Technik sind verschiedene Verabreichungsvorrichtungen bekannt. Das Dokument EP2247327B1 offenbart, eine Injektionsvorrichtung mit einer variablen Verbindung zwischen dem Stopfen und der Kolbenstange, welche ein Kolbenstangenmittel und einen Kolbenstangenfuss umfasst. Diese variable Verbindung dient dazu, dass die Kolbenstange und der Stopfen ohne Abstand zueinander zusammengebaut oder montiert werden können. Dies ist insbesondere wünschenswert, um einerseits Fertigungstoleranzen der Einzelteile der Injektionsvorrichtung auszugleichen und andererseits, dass der vor dem Verabreichungsvorgang durchgeführte Primingvorgang durch den Benutzer weggelassen werden kann. Der Primingvorgang ist vor dem Verabreichen einer Dosis durchzuführen, sodass eine korrekte Dosis verabreicht werden kann. Dies ist insbesondere bei einer Injektionsvorrichtung wichtig, bei welcher eine oder mehrere festgelegte und gleich grosse Dosen verabreicht werden.

Nachteilig bei dieser Injektionsvorrichtung ist, dass das Zusammenbauen oder das Montieren dieser Injektionsvorrichtung erschwert ist, da insbesondere mit einem aufwendigen Laserverfahren das Kolbenstangenmittel und der Kolbenstangenfuss miteinander verschweisst werden müssen, um eine Injektionsvorrichtung mit einer Kolbenstange zu bilden, welche mit dem Stopfen in einem Anschlagkontakt ist.

Es ist eine Aufgabe der Erfindung, eine Injektionsvorrichtung und ein Verfahren zum Zusammenbauen dieser Injektionsvorrichtung bereitzustellen, wobei das Zusammenbauen oder das Montieren der Injektionsvorrichtung und die Handhabung der Injektionsvorrichtung für den Benutzer vereinfacht sind.

Diese Aufgabe wird durch die Gegenstände der unabhängigen Ansprüche gelöst. Vorteilhafte Ausführungsformen sind in den abhängigen Ansprüchen definiert.

In die distale Richtung bedeutet bei der erfindungsgemässen Injektionsvorrichtung die Seite, an welcher eine Injektionsnadel angebracht sein kann, um das fluide Produkt zu verabreichen. In die proximale Richtung bedeutet bei der erfindungsmässen Injektionsvorrichtung die der distalen Seite entgegengesetzten Seite.

Die erfindungsgemässe Injektionsvorrichtung zur Verabreichung eines fluiden Produkts mit einer Längsachse (L) umfasst ein Gehäuse mit einem distalen und einem proximalen Ende. Das Gehäuse kann zumindest teilweise oder vollständig hülsenförmig ausgebildet sein.

Die Injektionsvorrichtung umfasst ferner einen Behälter mit dem fluiden Produkt und einen entlang der Längsachse (L) axial bewegbaren Stopfen. Der Stopfen ist in dem Behälter aufgenommen und kann in die distale Richtung bewegt werden, um das fluide Produkt auszuschütten. Der Stopfen kann über eine Kolbenstange in die distale Richtung bewegt werden. Die Kolbenstange ist mehrteilig, insbesondere mindestens zweiteilig ausgebildet. Die Kolbenstange kann somit auch mehr als zwei Teile umfassen. Die Kolbenstange ist zumindest teilweise oder vollständig in dem Gehäuse vorgesehen.

Die mindestens zweiteilige Kolbenstange umfasst ein Kolbenstangenmittel und einen Kolbenstangenfuss, wobei der Kolbenstangenfuss zumindest teilweise von dem Kolbenstangenmittel aufgenommen ist. Der Kolbenstangenfuss und das Kolbenstangenmittel sind relativ zueinander axial bewegbar und relativ zueinander drehfest angeordnet, wobei der axiale Abstand zwischen dem Kolbenstangenfuss und dem Stopfen während dem Zusammenbauen der Injektionsvorrichtung bestimmt wird und derart bestimmt wird, dass ein distales Ende des Kolbenstangenfusses und der Stopfen aneinander liegen. Durch die axiale Relativbewegung zwischen dem Kolbenstangenmittel und dem Kolbenstangenfuss kann die Länge der Kolbenstange bestimmt oder eingestellt werden. Während dem Zusammenbauen der Injektionsvorrichtung kann durch das Zusammenbringen des distalen Endes des Kolbenstangefusses mit dem Stopfen, insbesondere mit dem proximalen Ende des Stopfens erreicht werden, dass einerseits Fertigungstoleranzen der Einzelteile der Injektionsvorrichtung ausgeglichen werden und andererseits, dass der vor dem Verabreichungsvorgang durchgeführte Primingvorgang durch den Benutzer weggelassen werden kann. Nach dem Zusammenbringen des distalen Endes des Kolbenstangenfusses mit dem Stopfen, insbesondere mit dem proximalen Ende des Stopfens liegt eine Injektionsvorrichtung vor, durch welche ohne vorgängigen Primingvorgang eine korrekte Dosis verabreicht werden kann.

Die Injektionsvorrichtung weist einen Einsatz auf. Der Einsatz kann zumindest teilweise oder vollständig hülsenförmig ausgebildet sein. Der Einsatz weist ein Aussengewinde auf. Das Aussengewinde ist vorzugsweise an einer Aussenfläche des Einsatzes angeordnet. Der Einsatz ist an einem proximalen Ende der Kolbenstangenfusses und/oder innerhalb des Kolbenstangenfusses vorgesehen. Das Aussengewinde des Einsatzes ist in einem Gewindeeingriff mit einem Innengewinde an dem Kolbenstangenmittel oder alternativ mit einem Innengewinde an dem Kolbenstangenfuss, wobei bei relativer Drehung zwischen dem Einsatz und dem Kolbenstangenmittel oder alternativ dem Kolbenstangenfuss, der Kolbenstangenfuss relativ zu dem Kolbenstangenmittel axial bewegbar ist, sodass das distale Ende des Kolbenstangenfusses und der Stopfen aneinander liegen. Der Einsatz ist relativ zu dem Kolbenstangenmittel und/oder zu dem Kolbenstangenfuss drehbar angeordnet. Das Kolbenstangenmittel und/oder der Kolbenstangenfuss sind vorzugsweise drehfest mit dem Gehäuse verbunden. Der Einsatz der Injektionsvorrichtung bewirkt somit, dass während dem Zusammenbauen oder dem Montieren der Injektionsvorrichtung der Kolbenstangenfuss in die distale Richtung bewegt werden kann und dadurch in einen Anschlagkontakt mit dem Stopfen gelangt. Durch die Verwendung des Einsatzes der Injektionsvorrichtung kann das Zusammenbringen des distalen Endes des Kolbenstangenfusses mit dem Stopfen, insbesondere mit dem proximalen Ende des Stopfens möglichst einfach, kostengünstig und zuverlässig durchgeführt werden.

Der Kolbenstangenfuss ist von dem Kolbenstangenmittel zumindest teilweise aufgenommen. Der Kolbenstangenfuss ist relativ zu dem Kolbenstangenmittel axial bewegbar angeordnet. Somit kann die Länge der Kolbenstange bestimmt oder eingestellt werden. Das Kolbenstangenmittel ist vorzugsweise zumindest teilweise oder vollständig hülsenförmig ausgebildet. Der Kolbenstangenfuss kann aus Vollmaterial ausgebildet sein oder alternativ zumindest teilweise oder vollständig hülsenförmig ausgebildet sein. Der Kolbenstangenfuss ist vorzugsweise in dem zumindest teilweisen oder vollständig hülsenförmigen Kolbenstangenmittel aufgenommen.

In einer Ausführungsform der Erfindung kann an dem proximalen Ende des Kolbenstangenfusses eine Verzahnung und an einem distalen Ende des Einsatzes eine Verzahnung vorgesehen sein, wobei die beiden Verzahnungen relativ zueinander in eine erste Drehrichtung um die Längsachse (L) drehbar angeordnet sind. Die beiden Verzahnungen sind derart ausgebildet, dass die beiden Verzahnungen relativ zueinander in eine erste Drehrichtung um die Längsachse (L) drehbar sind. In dieser Ausführungsform ist der Einsatz mit dem Aussengewinde in dem Gewindeeingriff mit dem Innengewinde des Kolbenstangenmittels. Der Einsatz ist an dem proximalen Ende des Kolbenstangenfusses vorgesehen. Der Einsatz kann den Kolbenstangenfuss relativ zu dem Kolbenstangenmittel in die distale Richtung bewegen oder stossen. Der Einsatz ist relativ zu dem Kolbenstangenmittel axial bewegbar und drehbar angeordnet. Der Einsatz kann relativ zu dem Kolbenstangenmittel in die erste Drehrichtung um die Längsachse (L) gedreht werden, um den Kolbenstangenfuss in die distale Richtung zu verschieben.

In einer anderen Ausführungsform der Erfindung kann an dem proximalen Ende des Kolbenstangenmittel eine Verzahnung und an einem proximalen Ende des Einsatzes eine Verzahnung vorgesehen sein, wobei die beiden Verzahnungen relativ zueinander in eine erste Drehrichtung um die Längsachse (L) drehbar angeordnet sind. Die beiden Verzahnungen sind derart ausgebildet, dass die beiden Verzahnungen relativ zueinander in eine erste Drehrichtung um die Längsachse (L) drehbar sind. In dieser Ausführungsform ist der Einsatz mit dem Aussengewinde in dem Gewindeeingriff mit dem Innengewinde des Kolbenstangenfusses. Der Einsatz ist innerhalb des Kolbenstangenfusses vorgesehen ist. Vorzugsweise kann der Einsatz derart ausgebildet sein, dass er zumindest teilweise innerhalb des Kolbenstangenfusses vorgesehen ist und zudem an dem proximalen Ende des Kolbenstangenfusses angeordnet ist. Der Einsatz kann relativ zu dem Kolbenstangenfuss in die erste Drehrichtung um die Längsachse (L) gedreht werden, um den Kolbenstangenfuss in die distale Richtung zu verschieben.

Zudem können die beiden Verzahnungen der ersten und der anderen Ausführungsform der Erfindung derart ausgebildet sein, dass eine relative Drehung in eine zweite der ersten Drehrichtung entgegengesetzten Drehrichtung blockiert ist.

Durch den Gewindeeingriff zwischen dem Einsatz und dem Kolbenstangenmittel oder alternativ dem Kolbenstangenfuss wird durch die Drehung in die erste Drehrichtung um die Längsachse (L) bewirkt, dass der Kolbenstangenfuss relativ zu dem Kolbenstangenmittel axial bewegbar, insbesondere in die distale Richtung bewegbar ist, sodass das distale Ende des Kolbenstangenfusses und der Stopfen aneinander liegen. Der Einsatz wird dabei relativ zu dem Kolbenstangenmittel und/oder zu dem Kolbenstangenfuss in die erste Drehrichtung um die Längsachse (L) gedreht. Der Kolbenstangenfuss wird relativ zu dem Kolbenstangenmittel axial bewegt, insbesondere in die distale Richtung bewegt, bis das distale Ende des Kolbenstangenfusses mit dem Stopfen axial anschlägt, wobei das distale Ende des Kolbenstangenfusses und der Stopfen aneinander zu liegen kommen.

Während der relativen Drehung zwischen dem Einsatz und dem Kolbenstangenfuss oder alternativ dem Kolbenstangenmittel in die erste Drehrichtung um die Längsachse (L) kann die die Drehkraft zunehmen, um beim Erreichen eines bestimmten Schwellenwerts anzuzeigen, dass das distale Ende des Kolbenstangenfusses in einem axialen Anschlagkontakt mit dem Stopfen ist. Somit kann genau festgestellt werden, wann das distale Ende des Kolbenstangenfusses und der Stopfen, insbesondere das proximale Ende des Stopfens in einem axialen Anschlagkontakt sind. Bei diesem Schwellenwert ist die Verzahnung zwischen dem Einsatz und dem Kolbenstangenfuss oder alternativ dem Kolbenstangenmittel verrastet. Diese Verrastung zwischen dem Einsatz und dem Kolbenstangenfuss oder alternativ dem Kolbenstangenmittel bewirkt, dass das Kolbenstangenmittel und der Kolbenstangenfuss, insbesondere über den Einsatz relativ zueinander axialfest verbunden sind. Somit ist die mindestens zweiteilige Kolbenstange gebildet, welche den in dem Behälter aufgenommenen Stopfen in die distale Richtung bewegen kann, um das fluide Produkt auszuschütten.

Eine relative Drehung zwischen dem Einsatz und dem Kolbenstangenmittel und/oder dem Kolbenstangenfuss in eine zweite der ersten Drehrichtung entgegengesetzten Drehrichtung wird blockiert. Dazu sind die beiden Verzahnungen derart ausgebildet, dass eine relative Drehung in die erste Drehrichtung um die Längsachse (L) möglich ist und in die zweite Drehrichtung verhindert ist. Somit kann der Anschlagkontakt zwischen dem Kolbenstangenfuss und dem Stopfen nicht gelöst werden.

Der Einsatz kann mit einem Werkzeug der Injektionsvorrichtung in einen Eingriff, insbesondere in einen drehfesten Eingriff gelangen und das Werkzeug kann den Einsatz relativ zu dem Kolbenstangenmittel und/oder dem Kolbenstangenfuss in die erste Drehrichtung um die Längsachse (L) drehen. Der Einsatz kann insbesondere an dem proximalen Ende eine Öffnung aufweisen, welche dazu dient, dass das Werkzeug mit dem Einsatz in den Eingriff, insbesondere in den drehfesten Eingriff gelangen kann, um eine Drehung des Werkzeugs, insbesondere eine Drehung des Werkzeugs in die erste Drehrichtung um die Längsachse (L) auf den Einsatz zu übertragen.

Durch diese Drehung des Einsatzes oder des mit dem Werkzeug verbundenen Einsatzes kann der axiale Abstand zwischen dem Kolbenstangenfuss und dem Stopfen derart bestimmt werden, dass das distale Ende des Kolbenstangenfusses und der Stopfen aneinander zu liegen kommen. Dies ist ein einfacher, kostengünstiger und zuverlässiger Montageschritt, um das Zusammenbringen des distalen Endes des Kolbenstangenfusses mit dem Stopfen durchzuführen.

In einem Montageschritt kann der mit dem Behälter aufgenommenen Behälterhalter mit dem den Kolbenstangenfuss, das Kolbenstangenmittel und den Einsatz aufgenommenen Gehäuse axialfest verbunden werden. Es können eine Schnappverbindung, eine plastische Verformung, eine Schraubverbindung, eine Klebeverbindung oder eine Schweissverbindung vorgesehen sein.

In einer Ausführungsform der Erfindung wird in einem vorherigen Montageschritt der Einsatz mit einem Aussengewinde in das Kolbenstangenmittel mit einem Innengewinde geschraubt und der Kolbenstangenfuss wird durch das distale Ende des Kolbenstangenmittels in das Kolbenstangenmittel eingesetzt.

In einer anderen Ausführungsform der Erfindung wird alternativ in einem vorherigen Montageschritt der Einsatz mit einem Aussengewinde in den Kolbenstangenfuss mit einem Innengewinde geschraubt und der mit dem Einsatz aufgenommenen Kolbstangenfuss wird in das Kolbenstangenmittel eingesetzt.

Um eine feste, insbesondere axial- und drehfeste Verbindung zwischen dem Behälterhalter und dem Gehäuse zu bilden, kann der Behälterhalter ein oder mehrere Schnappelemente oder Schnappgegenelemente aufweisen, welche mit einem oder mit mehreren an dem Gehäuse vorgesehene Schnappgegenelement oder vorgesehenen Schnappelemente verschnappen. Die Schnappelemente und die Schnappgegenelemente können verschiedene Ausgestaltungen aufweisen.

Alternativ kann der Behälterhalter oder das Gehäuse ein oder mehrere plastisch verformbare Elemente, insbesondere eine oder mehrere plastisch verformbare Rippen aufweisen, welche in ein oder in mehrere korrespondierende Gegenelemente an dem Behälterhalter oder an dem Gehäuse, beispielsweise eine oder mehrere Mikroverzahnungen oder eine oder mehrere verzahnte Rippen eingreifen können, um eine feste, insbesondere axial- und drehfeste Verbindung zwischen dem Behälterhalter und dem Gehäuse zu bilden. Das plastisch verformbare Element und das korrespondierende Gegenelement können an einer Aussen- oder an einer Innenfläche des Behälteralters oder an einer Aussen- oder Innenfläche des Gehäuses vorgesehen sein, sodass durch eine relative axiale Bewegung und/oder durch eine relative Drehbewegung zwischen dem Behälterhalter und dem Gehäuse der Behälterhalter und das Gehäuse fest verbunden werden können, insbesondere axial- und drehfest verbunden werden können. Die oder die mehreren plastisch verformbaren Rippen sind aus einem plastisch verformbaren Material gebildet. Das oder die mehreren korrespondierenden Gegenelemente sind vorzugsweise aus einem starren oder aus keinem plastisch verformbaren Material oder einem weniger deformierbareren Material als das Material des plastisch verformbaren Elements gebildet.

Alternativ kann der Behälterhalter und das Gehäuse zusammen verschraubt, verklebt oder verschweisst, beispielsweise mittels Laserverschweissung verbunden werden.

Alternativ kann eine Schraubenmutter zwischen dem Behälterhalter und dem Gehäuse vorgesehen sein, um eine feste, insbesondere axial- und drehfeste Verbindung zwischen dem Behälterhalter und dem Gehäuse zu bilden. Die Schraubenmutter kann selbsthemmend ausbildet sein. Beispielsweise kann zuerst der Behälterhalter und das Gehäuse über eine Gewindeverbindung miteinander verbunden werden, wobei die Schraubenmutter zwischen dem Behälterhalter und dem Gehäuse angeordnet ist. Danach kann die Schraubenmutter die Gewindeverbindung zwischen dem Behälterhalter und dem Gehäuse sichern, indem durch eine relative Drehung zwischen der Schraubenmutter und dem Behältergehäuse oder dem Gehäuse eine an der Schraubenmutter vorgesehene Verzahnung in eine an dem Behälterhalter oder an dem Gehäuse vorgesehene Verzahnung eingreifen kann, um eine feste, insbesondere axial- und drehfeste Verbindung zwischen dem Behälterhalter und dem Gehäuse zu bilden.

Alternativ kann eine Hülse zwischen dem Behälterhalter und dem Gehäuse vorgesehen sein, um eine feste, insbesondere axial- und drehfeste Verbindung zwischen dem Behälterhalter und dem Gehäuse zu bilden. Die Hülse kann als Federelement oder zumindest teilweise federnd oder elastisch ausgebildet sein. Beispielsweise kann zuerst der Behälterhalter und das Gehäuse über eine Gewindeverbindung miteinander verbunden werden, wobei die Hülse zwischen dem Behälterhalter und dem Gehäuse angeordnet ist. Danach kann die Hülse die Gewindeverbindung zwischen dem Behälterhalter und dem Gehäuse sichern, indem durch eine relative Drehung zwischen der Hülse und dem Behältergehäuse oder dem Gehäuse eine an der Hülse vorgesehene Verzahnung in eine an dem Behälterhalter oder an dem Gehäuse vorgesehene Verzahnung eingreifen kann, um eine feste, insbesondere axial- und drehfeste Verbindung zwischen dem Behälterhalter und dem Gehäuse zu bilden. Alternativ kann die Hülse fest, insbesondere axial- und drehfest mit dem Behälterhalter oder dem Gehäuse verbunden sein oder einteilig mit dem Behälterhalter oder dem Gehäuse ausgebildet sein.

Es können auch Kombinationen aus den erwähnten Verbindungen vorgesehen sein.

Bevorzugt kann die Injektionsvorrichtung eine Behälterfixierung aufweisen, um den Behälter in dem Behälterhalter in die distale Richtung, insbesondere spielfrei zu halten. Die Behälterfixierung kann als Halteelement ausgebildet sein. Das Halteelement kann als separates Element ausgebildet sein oder alternativ mit dem Gehäuse, dem Behälterhalter oder dem Behälter der Injektionsvorrichtung einteilig ausgebildet sein. Das Halteelement kann zumindest teilweise hülsenförmig, vollständig hülsenförmig oder aus Vollmaterial ausgebildet sein. Das Halteelement kann vorzugsweise zumindest teilweise oder vollständig federnd oder elastisch ausgebildet sein. In einer anderen Ausführungsform der Erfindung kann das Halteelement vorzugsweise zumindest teilweise oder vollständig plastisch verformbar ausgebildet sein. Somit kann während dem Zusammenbauen des Behälterhalters mit dem Gehäuse das Halteelement den in dem Behälterhalter aufgenommenen Behälter relativ zu dem Behälterhalter in die distale Richtung bewegen oder drücken, sodass der Behälter in dem Behälterhalter spielfrei gehalten werden kann. Somit können Fertigungstoleranzen der Einzelteile der Injektionsvorrichtung ausgeglichen werden.

Das Halteelement kann vorzugsweise als Haltefeder oder als federndes Halteelement oder alternativ als plastisch verformbare Rippe oder Rippen ausgebildet sein, welche mit Behälter, insbesondere mit einem proximalen Rand des Behälters in einen Anschlagkontakt gelangen kann, um den Behälter relativ zu dem Behälterhalter in die distale Richtung zu schieben.

Es können auch Kombinationen aus den erwähnten Verbindungen vorgesehen sein.

Nachdem der Behälterhalter und das Gehäuse fest, insbesondere axial und drehfest miteinander verbunden sind, wird in einem weiteren Montageschritt der axiale Abstand zwischen dem Kolbenstangenfuss und dem Stopfen der Injektionsvorrichtung derart bestimmt, dass ein distales Ende des Kolbenstangenfusses und der Stopfen aneinander zu liegen kommt.

Durch die relative Drehung zwischen dem Einsatz und dem Kolbenstangenmittel oder alternativ dem Kolbenstangenfuss in die erste Drehrichtung um die Längsachse (L) wird der Kolbenstangenfuss relativ zu dem Kolbenstangenmittel axial verschoben, sodass das distale Ende des Kolbenstangenfusses und der Stopfen aneinander liegen. Beim Erreichen eines bestimmten Schwellenwerts kann durch Messung eines Kraftanstiegs, insbesondere eines sprunghaften Kraftanstiegs angezeigt werden, dass das distale Ende des Kolbenstangenfusses und der Stopfen aneinander liegen. Diese Schwellenwertdetektion misst einen Drehmomentanstieg, insbesondere einen sprunghaften Drehmomentanstieg, der bei der relativen Drehung in die erste Drehrichtung um die Längsachse (L) aufgrund der Verrastung zwischen der Verzahnung des Einsatzes und der Verzahnung des Kolbenstangenfusses oder der Verzahnung des Kolbenstangenmittels erfolgt. Diese Verrastung zwischen dem Einsatz und dem Kolbenstangenfuss oder alternativ dem Kolbenstangenmittel bewirkt, dass das Kolbenstangenmittel und der Kolbenstangenfuss, insbesondere über den Einsatz relativ zueinander axialfest verbunden sind. Somit ist die mindestens zweiteilige Kolbenstange gebildet, welche den in dem Behälter aufgenommenen Stopfen in die distale Richtung bewegen kann, um das fluide Produkt auszuschütten.

In einer anderen Ausführungsform der Erfindung kann alternativ eine Verzahnung an der Aussenfläche des Einsatzes vorgesehen sein, welche mit einem oder mehreren Schnappern an dem Kolbenstangenfuss oder an dem Kolbenstangenmittel eine Verrastung eingehen können, um eine axialfeste Verbindung zwischen dem Kolbenstangenmittel und dem Kolbenstangenfuss zu bilden, wobei das distale Ende des Kolbenstangenfusses und der Stopfen aneinander liegen. Der Schnapper kann federnd oder elastisch ausgebildet sein. Der Schnapper kann an einer Innenfläche an dem Kolbenstangenmittel oder an einer Innenfläche an dem Kolbenstangenfuss vorgesehen sein.

Die Erfindung wird im Folgenden an mehreren Figuren beschrieben. Die hierbei offenbarten Merkmale bilden je einzeln und in Kombination die Erfindung vorteilhaft weiter. Es zeigen:
- Figur 1:: eine Explosionsansicht einer ersten Ausführungsform der erfindungsgemässen Injektionsvorrichtung zur Verabreichung eines fluiden Produkts.
- Figur 2a: eine Längsschnittansicht eines Montageschritts der Injektionsvorrichtung gemäss Figur 1.
- Figur 2b: eine Längsschnittansicht eines weiteren Montageschritts der Injektionsvorrichtung gemäss Figur 1.
- Figur 2c: eine Längsschnittansicht eines weiteren Montageschritts der Injektionsvorrichtung gemäss Figur 1.
- Figur 3:: eine Explosionsansicht einer zweiten Ausführungsform der erfindungsgemässen Injektionsvorrichtung zur Verabreichung eines fluiden Produkts.
- Figur 4a: eine Längsschnittansicht eines Montageschritts der Injektionsvorrichtung gemäss Figur 3.
- Figur 4b: eine Längsschnittansicht eines weiteren Montageschritts der Injektionsvorrichtung gemäss Figur 3.
- Figur 4c: eine Längsschnittansicht eines weiteren Montageschritts der Injektionsvorrichtung gemäss Figur 3.
- Figur 5:: eine Explosionsansicht einer dritten Ausführungsform der erfindungsgemässen Injektionsvorrichtung zur Verabreichung eines fluiden Produkts.
- Figur 6a: eine Längsschnittansicht eines Montageschritts der Injektionsvorrichtung gemäss Figur 5.
- Figur 6b: eine Längsschnittansicht eines weiteren Montageschritts der Injektionsvorrichtung gemäss Figur 5.
- Figur 6c: eine Längsschnittansicht eines weiteren Montageschritts der Injektionsvorrichtung gemäss Figur 5.

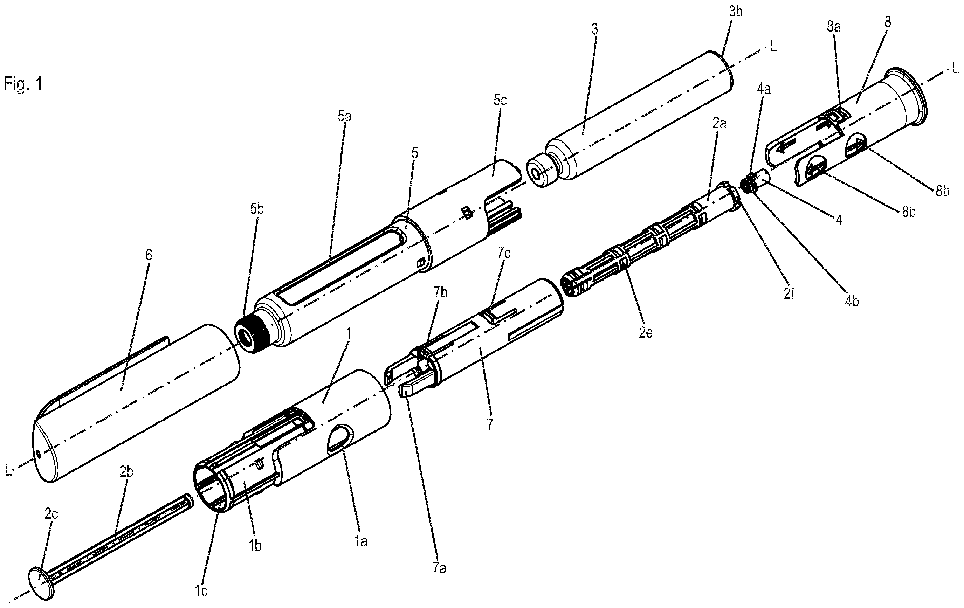

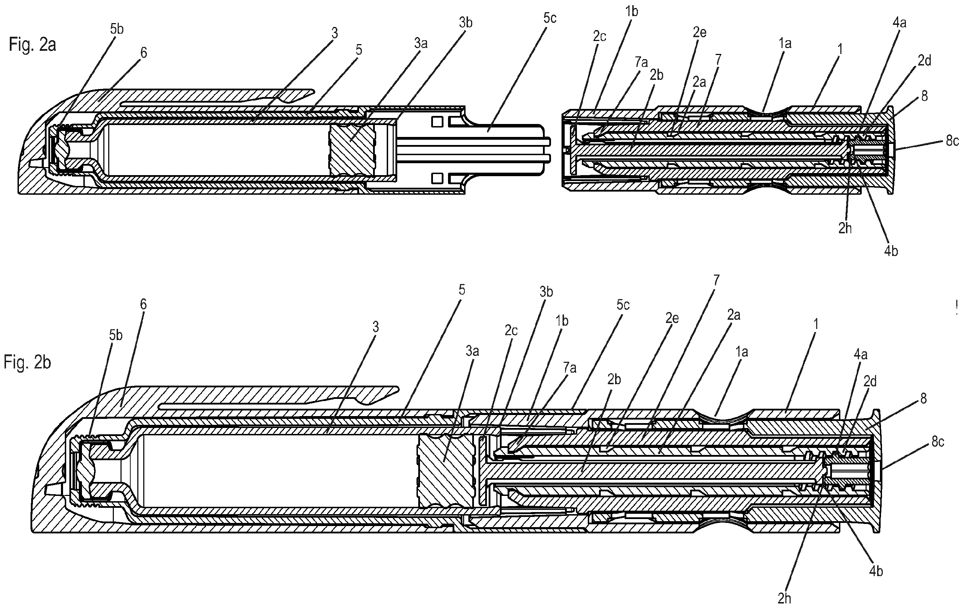

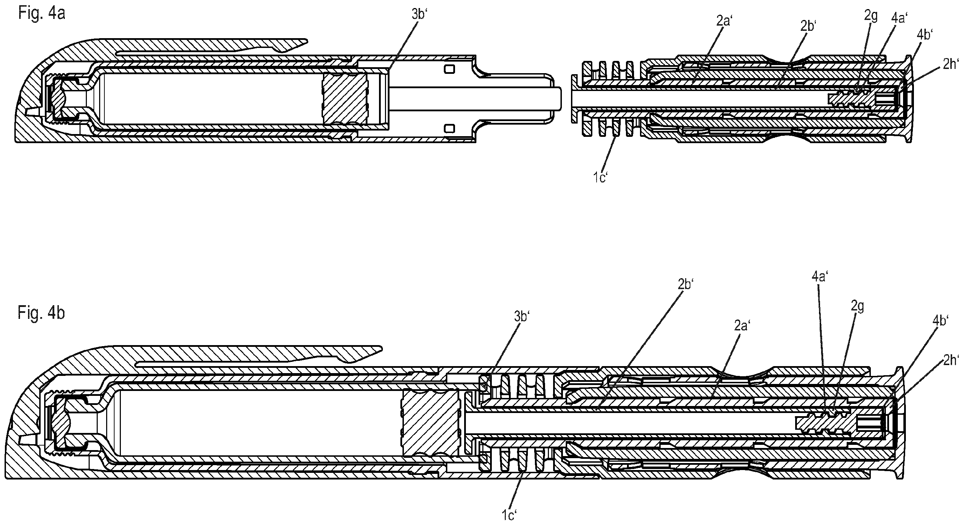

In der Figur 1 ist eine Explosionsansicht einer ersten Ausführungsform der erfindungsgemässen Injektionsvorrichtung zur Verabreichung eines fluiden Produkts dargestellt. Die Injektionsvorrichtung dient dazu, mehrere festgelegte und gleich grosse Dosen zu verabreichen. Die Injektionsvorrichtung mit einer Längsachse (L) umfasst ein Gehäuse (1) mit einem distalen und einem proximalen Ende. Das Gehäuse (1) ist hülsenförmig ausgebildet. Das Gehäuse (1) kann vorzugsweise ein Fenster (1a) aufweisen, durch welches beispielsweise angezeigt werden kann, ob eine festgelegte Dosis aufgezogen oder ausgeschüttet ist. Ferner umfasst die Injektionsvorrichtung eine Kolbenstange (2a; 2b), welche zumindest teilweise in dem Gehäuse (1) vorgesehen ist. Die Kolbenstange (2a; 2b) ist mehrteilig ausgebildet. Die Kolbenstange (2a; 2b) umfasst zumindest zwei Teile. Die mindestens zweiteilige Kolbenstange (2a; 2b) umfasst ein Kolbenstangenmittel (2a) und einen Kolbenstangenfuss (2b). Die Kolbenstange (2a; 2b) ist zumindest teilweise oder vollständig in dem Gehäuse (1) vorgesehen. Die Injektionsvorrichtung weist ferner einen Behälter (3) mit dem fluiden Produkt und einen entlang der Längsachse (L) axial bewegbaren Stopfen (3a; Fig. 2a) auf. Der Behälter (3) kann von einem Behälterhalter (5) aufgenommen sein. Der Behälter (3) kann in dem Behälterhalter (5) axial- und drehfest aufgenommen sein. Alternativ kann das Gehäuse (1) und der Behälterhalter (5) einteilig ausgebildet sein. Der Behälterhalter (5) kann vorzugsweise ein Fenster (5a) aufweisen, durch welches der Behälter (3), das fluide Produkt und/oder der bewegbare Stopfen (3a) ersichtlich sind. Das Fenster (5a) kann beispielsweise dazu dienen, dem Benutzer anzuzeigen, ob ein fluides Produkt noch intakt ist bzw. Trübungen oder Verfärbungen aufweist. Alternativ oder zusätzlich kann mit Hilfe des Fensters (5a) beispielsweise die Anzahl der verabreichten oder der noch zur Verfügung stehenden Dosen angezeigt werden. An dem distalen Ende des Behälterhalters (5) kann ein Aussengewinde (5b) vorgesehen sein, um eine wegnehmbare Injektionsnadel zu befestigen. Das distale Ende der Injektionsvorrichtung kann mit einer wegnehmbaren Verschlusskappe (6) abgedeckt werden. Die Kolbenstange (2a; 2b) kann den in dem Behälter (3) aufgenommenen Stopfen (3a) in die distale Richtung bewegen, um das fluide Produkt auszuschütten. Ein distales Ende der Kolbenstange (2a; 2b), insbesondere das distale Ende des Kolbenstangenfusses (2b) kann den Stopfen (3a) in die distale Richtung bewegen, um das fluide Produkt auszuschütten. An dem distalen Ende der Kolbenstange (2a; 2b), insbesondere an dem distalen Ende des Kolbenstangenfusses (2b) kann ein Flange (2c) vorgesehen sein, welcher den Stopfen (3a) in distale Richtung bewegen kann, um das fluide Produkt auszuschütten. Der Kolbenstangenfuss (2b) und der Flange (2c) sind axialfest, insbesondere axial- und drehfest verbunden. Der Kolbenstangenfuss (2b) und der Flange (2c) können einteilig, zweiteilig oder mehrteilig ausgebildet sein. Alternativ kann der Kolbenstangenfuss (2b) keinen Flange (2c) aufweisen. Der Kolbenstangenfuss (2b) ist zumindest teilweise von dem Kolbenstangenmittel (2a) aufgenommen, wobei der Kolbenstangenfuss (2b) und das Kolbenstangenmittel (2a) relativ zueinander axial bewegbar und relativ zueinander drehfest angeordnet sind. Der Kolbenstangenfuss (2b) ist vorzugsweise aus Vollmaterial und das Kolbenstangenmittel (2a) ist vorzugsweise hülsenförmig ausgebildet. Ferner umfasst die erste Ausführungsform der erfindungsgemässen Injektionsvorrichtung einen Einsatz (4), welcher an einem proximalen Ende des Kolbenstangenfusses (2b) vorgesehen ist, wobei der Einsatz (4) ein Aussengewinde (4a) aufweist, welches in einem Gewindeeingriff (4a; Figur 2a, 2d) mit einem Innengewinde (Figur 2a, 2d) an dem Kolbenstangenmittel (2a) ist. Der Einsatz (4) ist relativ zu dem Kolbenstangenmittel (2a) axial bewegbar und drehbar angeordnet. Der Einsatz (4) kann den Kolbenstangenfuss (2b) relativ zu dem Kolbenstangenmittel (4a) in die distale Richtung bewegen oder stossen. Bei relativer Drehung zwischen dem Einsatz (4) und dem Kolbenstangenmittel (2a) ist der Kolbenstangenfuss (2b) relativ zu dem Kolbenstangenmittel (2a) axial bewegbar, sodass das distale Ende des Kolbenstangenfusses (2b), insbesondere der Flansch (2c) des Kolbenstangenfusses (2b) und der Stopfen (3a) aneinander liegen oder in einen axialen Anschlagkontakt kommen. Der axiale Abstand zwischen dem Kolbenstangenfuss (2b), insbesondere zwischen dem Flansch (2c) des Kolbenstangenfusses (2b) und dem Stopfen (3a) wird während dem Zusammenbauen der Injektionsvorrichtung bestimmt. Ferner kann das proximale Ende des Kolbenstangenfusses (2b) vorzugsweise eine Verzahnung (Figur 2a, 2h) umfassen und das distale Ende des Einsatzes (4) kann vorzugsweise eine Verzahnung (4b) aufweisen, wobei die beiden Verzahnungen (Figur 2a, 2h; 4b) relativ zueinander in eine erste Drehrichtung um die Längsachse (L) drehbar angeordnet sind. Die beiden Verzahnungen (Figur 2a, 2h; 4b) können vorzugsweise derart ausgebildet sein, dass eine relative Drehung in eine zweite der ersten Drehrichtung entgegengesetzten Drehrichtung blockiert ist.

Die ersten Ausführungsform der erfindungsgemässen Injektionsvorrichtung weist ferner einen Dosisknopf (8) auf, welcher an dem proximalen Ende des Gehäuses (1) vorgesehen ist. Der Dosisknopf (8) kann relativ zu dem Gehäuse (1) axial hin und her bewegt werden, um eine festgelegte Dosis des fluiden Produkts aufzuziehen und aus dem Behälter (3) auszuschütten. Eine Markierung (8b), welche durch das Fenster (1a) in dem Gehäuse (1) ersichtlich ist, zeigt dem Benutzer an, ob eine Dosis aufgeladen oder ausgeschüttet ist. Um die Injektionsvorrichtung zu verwenden, nimmt der Benutzer die an dem distalen Ende der Injektionsvorrichtung vorgesehene Verschlusskappe (6) von der Injektionsvorrichtung weg und setzt eine Injektionsnadel auf ein an dem distalen Ende des Behälterhalters (5) vorgesehenes Aussengewinde (5b) der Injektionsvorrichtung. Der Benutzer zieht den Dosisknopf (8) in die proximale Richtung, um eine festgelegte Dosis zu laden. Der Dosisknopf (8) ist relativ zu dem Gehäuse (1) axial bewegbar, insbesondere axial bewegbar und drehfest angeordnet. Die erste Ausführungsform der erfindungsgemässen Injektionsvorrichtung umfasst ferner eine Vorschubhülse (7) mit einem Halteschnapper (7a), wobei die Vorschubhülse (7) zumindest teilweise oder vollständig in dem Gehäuse (1) vorgesehen ist. Zudem ist die Vorschubhülse (7) vorzugsweise relativ zu dem Gehäuse (1) drehfest angeordnet. Beim Laden der festgelegten Dosis wird die Vorschubhülse (7) aufgrund einer axialfesten Verbindung zwischen dem Dosisknopf (8) und der Vorschubhülse (7) in die proximale Richtung bewegt. Die axialfeste Verbindung zwischen dem Dosisknopf (8) und der Vorschubhülse (7) kann beispielsweise als Schnappverbindung zwischen einem Schnapper (7c) an der Vorschubhülse (7) und einer Aussparung (8a) im Dosisknopf (8) ausgebildet sein. In dieser ersten Ausführungsform der erfindungsgemässen Injektionsvorrichtung weist das Kolbenstangenmittel (2a) eine Zahnung (2e) auf. Der unter Kraftaufwand lösbare Halteschnapper (7a) der Vorschubhülse (7) wirkt derart mit der Zahnung (2e) des Kolbenstangenmittels (2a) zusammen, dass bei einer axialen Bewegung des Dosisknopfs (8) relativ zu dem Gehäuse (1) in die proximale Richtung der Halteschnapper (7a) der Vorschubhülse (7) über die Zahnung (2e) des Kolbenstangenmittels (2a) in die proximale Richtung gleitet. Das Kolbenstangenmittel (2a) wird dabei relativ zu dem Gehäuse (1) axialfest gehalten. Dazu weist das Gehäuse (1) einen Halteschnapper (nicht ersichtlich) auf, welcher derart mit der Zahnung (2e) des Kolbenstangenmittels (2a) zusammenwirkt, dass bei der axialen Bewegung des Dosisknopfs (8) relativ zu dem Gehäuse (1) in die proximale Richtung der Halteschnapper (nicht ersichtlich) des Gehäuses (1) das Kolbenstangenmittel (2a) relativ zu dem Gehäuse (1) axialfest hält. Beim Ausschütten der festgelegten Dosis drückt der Benutzer den Dosisknopf (8) in die distale Richtung. Aufgrund der axialfesten Verbindung zwischen dem Dosisknopf (8) und der Vorschubhülse (7) wird die Vorschubhülse (7) in die distale Richtung bewegt. Der Halteschnapper (7a) der Vorschubhülse (7) wirkt derart mit der Zahnung (2e) des Kolbenstangenmittels (2a) zusammen, dass bei der axialen Bewegung des Dosisknopfs (8) relativ zu dem Gehäuse (1) in die distale Richtung der Halteschnapper (7a) der Vorschubhülse (7) das Kolbenstangenmittel (2a) mit Hilfe der Zahnung (2e) des Kolbenstangemittels (2a) in einem Eingriff hält, sodass die Vorschubhülse (7) das Kolbenstangenmittel (2a) in die distale Richtung mitnimmt. Dabei wird das Kolbenstangenmittel (2a) relativ zu dem Gehäuse (1) in die distale Richtung bewegt. Aufgrund der Verrastung der beiden Verzahnungen (Figur 2a, 4b; 2h) zwischen dem Einsatz (4) und dem Kolbenstangenfuss (2b) sind das Kolbenstangenmittel (2a) und der Kolbenstangenfuss (2b) relativ zueinander axialfest, insbesondere axial- und drehfest verbunden. Das Kolbenstangenmittel (2a) bewegt somit über den Kolbenstangenfuss (2b), insbesondere über den mit dem Kolbenstangenfuss (2b) axial verbundenen Flansch (2c) den Stopfen (3a) in die distale Richtung, um das fluide Produkt auszuschütten. Dabei wirkt der unter Kraftaufwand lösbare Halteschnapper (nicht ersichtlich) des Gehäuses (1) derart mit der Zahnung (2e) des Kolbenstangenmittels (2a) zusammen, dass bei einer axialen Bewegung des Dosisknopfs (8) relativ zu dem Gehäuse (1) in die distale Richtung der Halteschnapper (nicht ersichtlich) des Gehäuses (1) über die Zahnung (2e) des Kolbenstangenmittels (2a) gleitet und in die nachfolgende Zahnung (2e) des Kolbenstangenmittels (2a), welche proximal zu der vorangehenden Zahnung (2e) an dem Kolbenstangenmittel (2a) angeordnet ist, eingreift. Dabei wird das Kolbenstangenmittel (2a) relativ zu dem Gehäuse (1) in die distale Richtung bewegt und schüttet die festgelegte Dosis aus. Die festgelegte Dosis wird durch die entsprechenden axialen Anschlagkontakte zwischen einem distalen und einem proximalen nach innen ragenden Vorsprung (nicht ersichtlich) an dem Gehäuse (1) und einer nach aussen ragenden Nocke (7b) an der Vorschubhülse (7) definiert.

Um eine erneute festgelegt Dosis zuladen, zieht der Benutzer den Dosisknopf (8) erneut in die proximale Richtung. Am Ende der letzten ausgeschütteten Dosis ist ein an der Vorschubhülse (7) vorgesehener Anschlag (7c) in einem Anschlagkontakt mit einer an dem proximalen Ende oder in einem proximalen Bereich des Kolbenstangenmittels (2a) vorgesehenen Anschlagschulter (2f). Eine weitere festgelegte Dosis kann somit nicht geladen werden.

In der Figur 2a ist eine Längsschnittansicht eines ersten Montageschritts der ersten Ausführungsform der erfindungsgemässen Injektionsvorrichtung gemäss Figur 1 dargestellt.

In einem Montageschritt wird eine Behälterhaltereinheit gebildet. Zur Bildung der Behälterhaltereinheit kann zuerst die Verschlusskappe (6) auf den Behälterhalter (5) gesetzt werden. Alternativ kann die Verschlusskappe (6) während einem anderen Montageschritt auf den Behälterhalter (5) gesetzt werden. In einem weiteren Montageschritt kann der mit dem fluiden Produkt vorgefüllte Behälter (3) in den Behälterhalter (5) geschoben werden. Der mit dem Stopfen (3a) aufgenommenen Behälter (3) wird dabei durch ein proximales Ende des Behälterhalters (5) relativ zu dem Behälterhalter (5) in die distale Richtung verschoben. In einer anderen Ausführungsform der erfindungsgemässen Injektionsvorrichtung kann vor dem Einsetzen des Behälters (3) in den Behälterhalter (5) eine Behälterfixierung, beispielsweise in Form eines Halteelements (1c) zum spielfreien Halten des Behälters (3) in die distale Richtung in den Behälterhalter (5) eingesetzt werden. In dieser ersten Ausführungsform der erfindungsgemässen Injektionsvorrichtung ist das Halteelement (1c) in Form einer plastisch verformbaren Rippe ausgebildet.

In einem weiteren Montageschritt wird eine Antriebseinheit gebildet. Zur Bildung der Antriebseinheit wird zuerst das Kolbenstangenmittel (2a) in die Vorschubhülse (7) gesetzt. Das Kolbenstangenmittel (2b) wird dabei durch das proximale Ende der Vorschubhülse (7) geschoben und relativ zu der Vorschubhülse (7) in die distale Richtung verschoben. In einem weiteren Montageschritt wird der Einsatz (4) über die Gewindeverbindung (2d; 4a) zwischen dem Innengewinde (2d) des Kolbenstangenmittels (2a) und dem Aussengewinde (4a) des Einsatzes (4) in das Kolbenstangenmittel (2a) eingeschraubt. Der Einsatz (4) wird dabei durch das proximale Ende des Kolbenstangenmittels (2a) in die distale Richtung in das Kolbenstangenmittel (2b) eingeschraubt. In einem weiteren Montageschritt wird der Dosisknopf (8) auf das proximale Ende der mit dem Kolbenstangenmittel (2a) aufgenommenen Vorschubhülse (7) aufgesetzt und relativ zu der Vorschubhülse (7) in die distale Richtung verschoben. In einem weiteren Montageschritt wird die Baueinheit bestehend aus dem Dosisknopf (8), der Vorschubhülse (7), dem Einsatz (4) und dem Kolbenstangenmittel (2a) in das Gehäuse (1) gesetzt. Diese Baueinheit wird dabei durch das proximale Ende des Gehäuses (1) geschoben und relativ zu dem Gehäuse (1) in die distale Richtung verschoben. In einem weiteren Montageschritt wird der Kolbenstangenfuss (2b) in das Kolbenstangenmittel (2a) gesetzt. Der Kolbenstangenfuss (2b) wird dabei durch das distale Ende des Kolbenstangenmittels (2b) geschoben und relativ zu dem Kolbenstangenmittel (2b) in die proximale Richtung verschoben. In einer anderen Ausführungsform der erfindungsgemässen Vorrichtung wird der Flange (2c) vor dem Einsetzen des Kolbenstangenfusses (2b) in das Kolbenstangenmittel (2) an den Kolbenstangenfuss (2b) axialfest befestigt. In einer weiteren Ausführungsform der erfindungsgemässen Injektionsvorrichtung wird der Flange (2c) nach dem Einsetzen des Kolbenstangenfusses (2b) in das Kolbenstangenmittel (2) an den Kolbenstangenfuss (2b) axialfest befestigt. In dieser ersten Ausführungsform der erfindungsgemässen Vorrichtung ist der Flange (2c) einteilig mit dem Kolbenstangenfuss (2b) ausgebildet.

In einem weiteren Montageschritt werden die Behälterhaltereinheit und die Antriebseinheit zusammengefügt, wie in der Figur 2b ersichtlich ist. In diesem Montageschritt wird der mit dem Behälter (3) aufgenommenen Behälterhalter (5) mit dem den Kolbenstangenfuss (2b), das Kolbenstangenmittel (2a) und den Einsatz (4) aufgenommenen Gehäuse (1) axialfest verbunden. Es können eine Schnappverbindung, eine plastische Verformung, eine Schraubverbindung, eine Klebeverbindung oder eine Schweissverbindung vorgesehen sein.

In dieser ersten Ausführungsform der erfindungsgemässen Vorrichtung ist an dem Behälterhalter (5) ein Verbindungselement (5c) vorgesehen, welches mit einem Verbindungselement (1b) des Gehäuses (1) verbunden werden kann. Das Verbindungselement (5c) des Behälterhalters (5) oder das Verbindungselement (1b) des Gehäuses (1) können ein oder mehrere plastisch verformbare Elemente, insbesondere eine oder mehrere plastisch verformbare Rippen aufweisen, welche in ein oder in mehrere korrespondierende Gegenelemente an dem Verbindungselement (1b) des Behälterhalters (5) oder an dem Verbindungselement (1b) des Gehäuses (1), beispielsweise eine oder mehrere Mikroverzahnungen oder eine oder mehrere verzahnte Rippen eingreifen können, um eine feste, insbesondere axial- und drehfeste Verbindung zu bilden.

Die oder die mehreren plastisch verformbaren Rippen können aus einem plastisch verformbaren Material gebildet sein. Das oder die mehreren korrespondierenden Gegenelemente können vorzugsweise aus einem starren oder aus keinem plastisch verformbaren Material oder einem weniger deformierbareren Material als das Material des plastisch verformbaren Elements gebildet sein.

Bei dem Zusammenfügen der Behälterhaltereinheit mit der Antriebseinheit, gelangt das Halteelement (1c) in einen Anschlagkontakt mit dem proximalen Rand (3b) des Behälters (3). Dadurch wird der Behälter (3) relativ zu dem Behälterhalter (5) in die distale Richtung gedrückt, um den Behälter (3) in dem Behälterhalter (5) spielfrei zu halten.

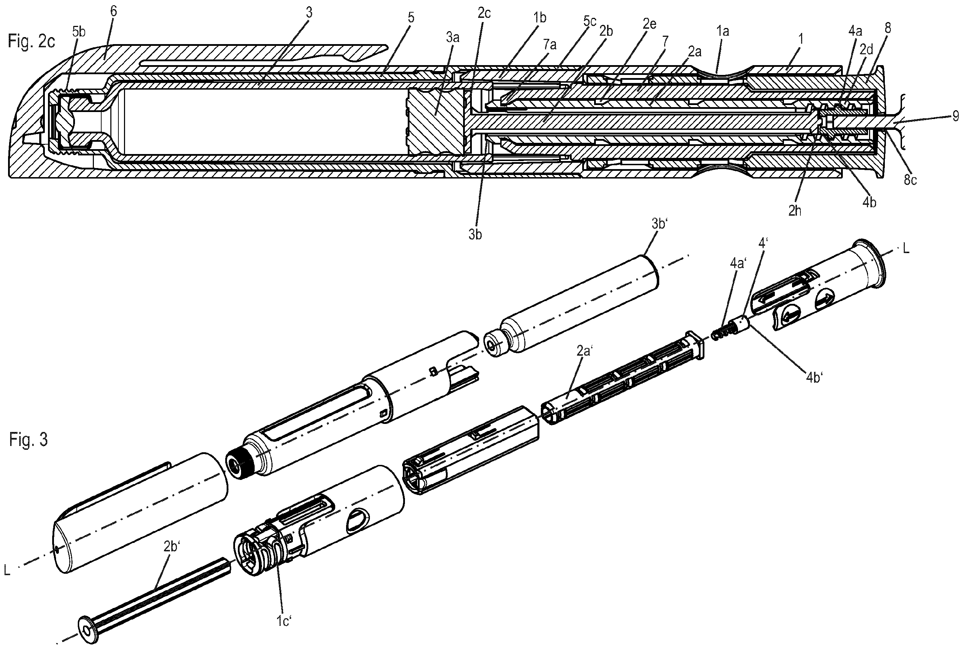

In einem weiteren Montageschritt, wie in der Figur 2c gezeigt ist, wird der axiale Abstand zwischen dem Kolbenstangenfuss (2b), insbesondere der axiale Abstand zwischen dem mit dem Kolbenstangenfuss (2b) axialfest verbundenen Flange (2c) und dem Stopfen (3a) der Injektionsvorrichtung derart bestimmt, dass das distale Ende des Kolbenstangenfusses (2b), insbesondere der mit dem Kolbenstangenfuss (2b) axialfest verbundene Flange (2c) und der Stopfen (3a) aneinander zu liegen kommt.

Ein Werkzeug (9) wird durch eine Öffnung (8c) an dem proximalen Ende des Dosisknopfs (8) in den Einsatz (4) geschoben. Der Einsatz (4) weist insbesondere eine Öffnung an dem proximalen Ende auf. Das Werkzeug (9) gelangt in einen Eingriff, insbesondere in einen drehfesten Eingriff mit dem Einsatz (4). Das Werkzeug (9) dreht den Einsatz (4) relativ zu dem Kolbenstangenmittel (2a) aufgrund der Gewindeverbindung (4a; 2d) zwischen dem Einsatz (4) und dem Kolbenstangenmittel (2a) in die erste Drehrichtung um die Längsachse (L). Die Drehung des Werkzeugs (9) in die erste Drehrichtung um die Längsachse (L) wird auf den Einsatz (4) übertragen.

Durch diese Drehung des Einsatzes (4) oder des mit dem Werkzeug (9) verbundenen Einsatzes (4) kann der axiale Abstand zwischen dem Kolbenstangenfuss (2b), insbesondere der axiale Abstand zwischen dem mit dem Kolbenstangenfuss (2b) axialfest verbundenen Flange (2c) und dem Stopfen (3a) derart bestimmt werden, dass das distale Ende des Kolbenstangenfusses (2b), insbesondere der mit dem Kolbenstangenfuss (2b) axialfest verbundene Flange (2c) und der Stopfen aneinander zu liegen kommen. Der Kolbenstangenfuss (2b) wird innerhalb des Kolbenstangenmittels (2a) durch die kombinierte Axial- und Drehbewegung des Einsatzes (4) und einem Anschlagkontakt zwischen dem Einsatz (4) und dem Kolbenstangenfuss (2b) relativ zu dem Kolbenstangenmittel (2a) in die distale Richtung bewegt oder verschoben. Das Kolbenstangenmittel (2a) wird aufgrund des Eingriffs zwischen dem Halteschnapper (7a) der Vorschubhülse (7) und der Zahnung (2h) des Kolbenstangenmittels (2a) und aufgrund des Eingriffs zwischen dem Halteschnapper (nicht ersichtlich) des Gehäuses (1) und der Zahnung (2h) des Kolbenstangenmittels (2a) relativ zu dem Gehäuse (1) axialfest gehalten. Während der relativen Drehung zwischen dem Einsatz (4) und dem Kolbenstangenmittel (2a) in die erste Drehrichtung um die Längsachse (L) nimmt die Drehkraft zu, um beim Erreichen eines bestimmten Schwellenwerts anzuzeigen, dass das distale Ende des Kolbenstangenfusses (2b), insbesondere der mit dem Kolbenstangenfuss (2b) axialfest verbundene Flange (2c) in einem axialen Anschlagkontakt mit dem Stopfen (3a) ist. Ein Drehmomentanstieg, insbesondere ein sprunghafter Drehmomentanstieg, welcher detektiert werden kann, zeigt an, dass das distale Ende des Kolbenstangenfusses (2b), insbesondere der mit dem Kolbenstangenfuss (2b) axialfest verbundene Flange (2c) in einem axialen Anschlagkontakt mit dem Stopfen (3a) ist. Somit kann genau festgestellt werden, wann das distale Ende des Kolbenstangenfusses (2b), insbesondere der mit dem Kolbenstangenfuss (2b) axialfest verbundene Flange (2c) und der Stopfen (3a) in einem axialen Anschlagkontakt sind. Bei diesem Schwellenwert ist die Verzahnung (4b; 2h) zwischen dem Einsatz (4) und dem Kolbenstangenfuss (2b) verrastet. Diese Verrastung zwischen dem Einsatz (4) und dem Kolbenstangenfuss (2b) bewirkt, dass das Kolbenstangenmittel (2a) und der Kolbenstangenfuss (2b), insbesondere über den Einsatz (4) relativ zueinander axialfest verbunden sind. Somit ist die mindestens zweiteilige Kolbenstange (2a; 2b) gebildet, welche den in dem Behälter (3) aufgenommenen Stopfen (3a) in die distale Richtung bewegen kann, um das fluide Produkt auszuschütten.

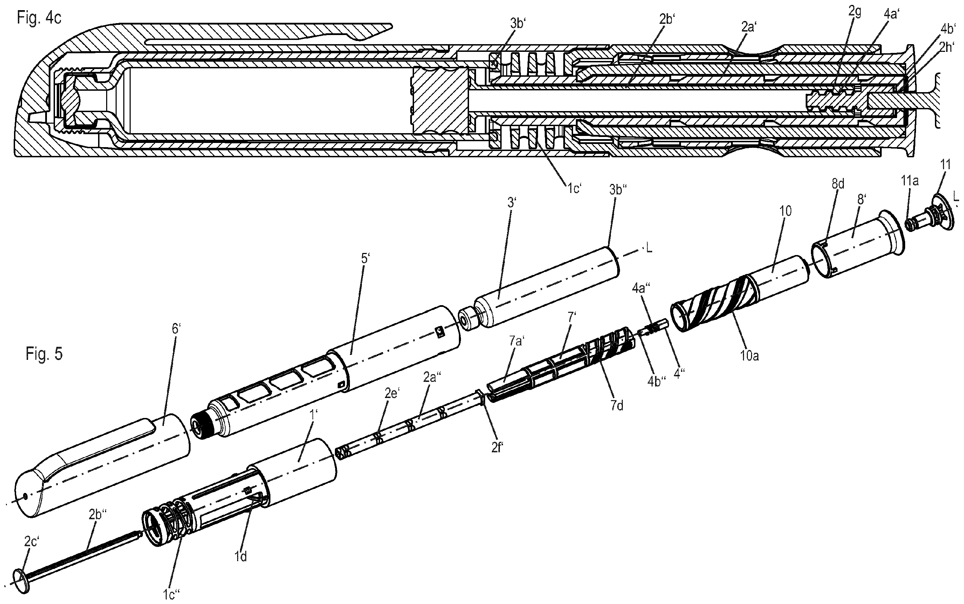

In einem weiteren Montageschritt kann die Öffnung (8c) an dem proximalen Ende des Dosisknopfs (8) beispielsweise mit einer Etikette oder einer Dosiskappe (11), wie beispielsweise in dem dritten Ausführungsbeispiel der erfindungsgemässen Injektionsvorrichtung in der Figur 5 gezeigt ist, verschlossen werden.

In der Figur 3 ist eine Explosionsansicht einer zweiten Ausführungsform der erfindungsgemässen Injektionsvorrichtung zur Verabreichung eines fluiden Produkts dargestellt. Die erste und die zweite Ausführungsform der erfindungsgemässen Injektionsvorrichtung unterscheiden sich zueinander durch die Anordnung des Einsatzes (3') und den daraus resultierenden Anpassungen der Ausgestaltung des Kolbenstangenmittels (2a') und des Kolbenstangenfusses (2b'). Zudem weist die Behälterfixierung eine andere Ausgestaltung auf.

Die zweite Ausführungsform der Injektionsvorrichtung umfasst einen Einsatz (4'), welcher innerhalb des Kolbenstangenfusses (2b') vorgesehen ist, wobei der Einsatz (4') ein Aussengewinde (4a') aufweist, welches in einem Gewindeeingriff (4a'; Figur 4a, 2g) mit einem Innengewinde (Figur 4a, 2g) an dem Kolbenstangenfusses (2b') ist.

Der Einsatz (4') ist relativ zu dem Kolbenstangenfuss (2b') axial bewegbar und drehbar angeordnet. Bei relativer Drehung zwischen dem Einsatz (4') und dem Kolbenstangenfuss (2b') ist der Kolbenstangenfuss (2b') relativ zu dem Kolbenstangenmittel (4a') axial bewegbar, sodass das distale Ende des Kolbenstangenfusses (2b'), insbesondere der Flansch des Kolbenstangenfusses (2b') und der Stopfen aneinander liegen oder in einen axialen Anschlagkontakt kommen. Der axiale Abstand zwischen dem Kolbenstangenfuss (2b'), insbesondere zwischen dem Flansch des Kolbenstangenfusses (2b') und dem Stopfen wird während dem Zusammenbauen der Injektionsvorrichtung bestimmt. Der Einsatz (4') kann relativ zu dem Kolbenstangenfusses (2b') in die erste Drehrichtung um die Längsachse (L) gedreht werden, um den Kolbenstangenfuss (2b') relativ zu dem Kolbenstangenmittel (4a') in die distale Richtung zu verschieben. Ferner kann das proximale Ende des Kolbenstangenmittels (2a') vorzugsweise eine Verzahnung (2h') umfassen und das proximale Ende des Einsatzes (4') kann vorzugweise eine Verzahnung (4b') aufweisen, wobei die beiden Verzahnungen (4b'; Figur 4a, 2h') relativ zueinander in eine erste Drehrichtung um die Längsachse (L) drehbar angeordnet sind. Die beiden Verzahnungen (4b'; Figur 4a, 2h') können vorzugsweise derart ausgebildet sein, dass eine zweite der ersten Drehrichtung entgegengesetzten Drehrichtung blockiert ist.

In der Figur 4a ist eine Längsschnittansicht eines ersten Montageschritts der zweiten Ausführungsform der erfindungsgemässen Injektionsvorrichtung gemäss Figur 3 dargestellt.

Die Montageschritte der erste und der zweiten Ausführungsform der erfindungsgemässen Injektionsvorrichtung unterscheiden sich zueinander durch einzelne Montageschritte, welche durch die andere Anordnung des Einsatzes (4') in der Injektionsvorrichtung resultieren.

In einem Montageschritt wird eine Behälterhaltereinheit gebildet. Die Behälterhaltereinheit umfasst wie die Behälterhaltereinheit gemäss der ersten Ausführungsform der erfindungsgemässen Injektionsvorrichtung die Verschlusskappe und den mit dem Stopfen aufgenommenen Behälter, wobei der Behälter in den Behälterhalter eingesetzt ist.

In einem weiteren Montageschritt wird eine Antriebseinheit gebildet. Die Bildung der Antriebseinheit gemäss der zweiten Ausführungsform der erfindungsgemässen Injektionsvorrichtung unterscheidet sich zu den Montageschritten der Antriebseinheit gemäss der ersten Ausführungsform der erfindungsgemässen Injektionsvorrichtung dadurch, dass der Einsatz (4') mit dem Aussengewinde (4a') in ein Innengewinde (2g) des Kolbenstangenfusses (2b') anstatt in ein Innengewinde (2d) des Kolbenstangenmittels (2a) eingeschraubt wird.

Das Kolbenstangenmittel wird in einem ersten Montageschritt zuerst in eine Vorschubhülse in die distale Richtung geschoben. Der Dosisknopf wird dann über die Vorschubhülse in distale Richtung geschoben. Danach wird die Baueinheit bestehend aus dem Dosisknopf, der Vorschubhülse und des Kolbenstangenmittels in das Gehäuse in die distale Richtung geschoben.

Der mit dem Einsatz (4') aufgenommenen Kolbenstangenfuss (2b') wird in einem weiteren Montageschritt in das Kolbenstangenmittel (2a') gesetzt. Der mit dem Einsatz (4') aufgenommenen Kolbenstangenfuss (2b') wird dabei durch das distale Ende des Kolbenstangenmittels (2b) geschoben und relativ zu dem Kolbenstangenmittel (2b') in die proximale Richtung axial verschoben.

In einem weiteren Montageschritt werden die Behälterhaltereinheit und die Antriebseinheit zusammengefügt, wie in der Figur 4b ersichtlich ist.

In diesem Montageschritt wird der mit dem Behälter aufgenommenen Behälterhalter mit dem den Kolbenstangenfuss (2b'), das Kolbenstangenmittel (2a') und den Einsatz (4') aufgenommenen Gehäuse axialfest verbunden. Es können eine Schnappverbindung, eine plastische Verformung, eine Schraubverbindung, eine Klebeverbindung oder eine Schweissverbindung vorgesehen sein.

In dieser zweiten Ausführungsform der erfindungsgemässen Vorrichtung ist an dem Gehäuse ein Halteelement (1c') in Form einer Haltefeder vorgesehen. Die Haltefeder ist fest, insbesondere axial- und drehfest, besonders bevorzugt einteilig mit dem Gehäuse verbunden. Das Halteelement (1c') gelangt beim Zusammenfügen der Behälterhaltereinheit mit der Antriebseinheit in einen Anschlagkontakt mit einem proximalen Rand (3b') des Behälters, um den Behälter relativ zu dem Behälterhalter spielfrei in die distale Richtung zu drücken.

In einem weiteren Montageschritt, wie in der Figur 4c gezeigt ist, wird der axiale Abstand zwischen dem Kolbenstangenfuss (2b'), insbesondere der axiale Abstand zwischen dem mit dem Kolbenstangenfuss (2b') axialfest verbundenen Flange und dem Stopfen der Injektionsvorrichtung derart bestimmt, dass ein distales Ende des Kolbenstangenfusses (2b'), insbesondere der mit dem Kolbenstangenfuss (2b') axialfest verbundene Flange und der Stopfen aneinander zu liegen kommt.

Ein Werkzeug wird durch die Öffnung an dem proximalen Ende des Dosisknopfs in den Einsatz (4') geschoben. Das Werkzeug gelangt in einen Eingriff, insbesondere in einen drehfesten Eingriff mit dem Einsatz (4'). Das Werkzeug der Injektionsvorrichtung dreht den Einsatz (4') relativ zu dem Kolbenstangenfuss (2b') aufgrund der Gewindeverbindung (4a'; 2g) zwischen dem Einsatz (4') und dem Kolbenstangenfuss(2b') in die erste Drehrichtung um die Längsachse (L). Die Drehung des Werkzeugs in die erste Drehrichtung um die Längsachse (L) wird auf den Einsatz (4') übertragen. Das Werkzeug dient ferner dazu, dass die Verzahnung (4b') des Einsatzes (4') mit der Verzahnung (2h') des Kolbenstangenmittels (2a') in einem Eingriff gehalten werden können, sodass bei der Drehung des Werkzeugs in die erste Drehrichtung um die Längsachse (L) der Einsatz (4') um die Höhe der Verzahnung relativ zu dem Kolbenstangenmittel (2a') oder/und relativ zu dem Gehäuse axial bewegbar ist. Durch diese Drehung in die erste Drehrichtung um die Längsachse (L) des Einsatzes (4) oder des mit dem Werkzeug verbundenen Einsatzes (4) kann der axiale Abstand zwischen dem Kolbenstangenfuss (2b'), insbesondere zwischen dem mit dem Kolbenstangenfuss (2b') axialfest verbundenen Flange und dem Stopfen (3a) derart bestimmt werden, dass das distale Ende des Kolbenstangenfusses (2b'), insbesondere der mit dem Kolbenstangenfuss (2b') axialfest verbundene Flange und der Stopfen aneinander zu liegen kommen. Der Kolbenstangenfuss (2b') wird innerhalb des Kolbenstangenmittels (2a') aufgrund der Drehbewegung des Einsatzes (4), dem Gewindeeingriff (4a'; 2g) zwischen dem Kolbenstangenfusses (2b') und dem Kolbenstangenmittels (2a') und der axialfesten Verbindung zwischen dem Einsatz (4') und dem Kolbenstangenmittels (2a') relativ zu dem Kolbenstangenmittel (2a') in die distale Richtung bewegt oder verschoben. Während der relativen Drehung zwischen dem Einsatz (4') und dem Kolbenstangenfuss (2b') in die erste Drehrichtung um die Längsachse (L) nimmt die die Drehkraft zu, um beim Erreichen eines bestimmten Schwellenwerts anzuzeigen, dass das distale Ende des Kolbenstangenfusses (2b'), insbesondere der mit dem Kolbenstangenfuss (2b') axialfest verbundene Flange in einen axialen Anschlagkontakt mit dem Stopfen (3a) ist. Ein Drehmomentanstieg, insbesondere ein sprunghafter Drehmomentanstieg, welcher detektiert werden kann, zeigt an, dass das distale Ende des Kolbenstangenfusses (2b'), insbesondere der mit dem Kolbenstangenfuss (2b') axialfest verbundene Flange in einem axialen Anschlagkontakt mit dem Stopfen ist. Somit kann genau festgestellt werden, wann das distale Ende des Kolbenstangenfusses (2b'), insbesondere der mit dem Kolbenstangenfuss (2b') axialfest verbundene Flange und der Stopfen (3a) in einem axialen Anschlagkontakt sind. Bei diesem Schwellenwert ist die Verzahnung (4b'; 2h') zwischen dem Einsatz (4') und dem Kolbenstangenfuss (2b') verrastet. Diese Verrastung zwischen dem Einsatz (4') und dem Kolbenstangenfuss (2b') bewirkt, dass das Kolbenstangenmittel (2a') und der Kolbenstangenfuss (2b'), insbesondere über den Einsatz (4') relativ zueinander axialfest verbunden sind. Somit ist die mindestens zweiteilige Kolbenstange (2a'; 2b') gebildet, welche den in dem Behälter aufgenommenen Stopfen in die distale Richtung bewegen kann, um das fluide Produkt auszuschütten.

In der Figur 5 ist eine Explosionsansicht einer dritten Ausführungsform der erfindungsgemässen Injektionsvorrichtung zur Verabreichung eines fluiden Produkts dargestellt. Die erste und die dritte Ausführungsform der erfindungsgemässen Injektionsvorrichtung unterscheiden sich zueinander durch die Funktion der Injektionsvorrichtung, nämlich durch die Funktion des Ladens und des Ausschüttens einer festgelegten Dosis. Zudem weist die Behälterfixierung eine andere Ausgestaltung auf.

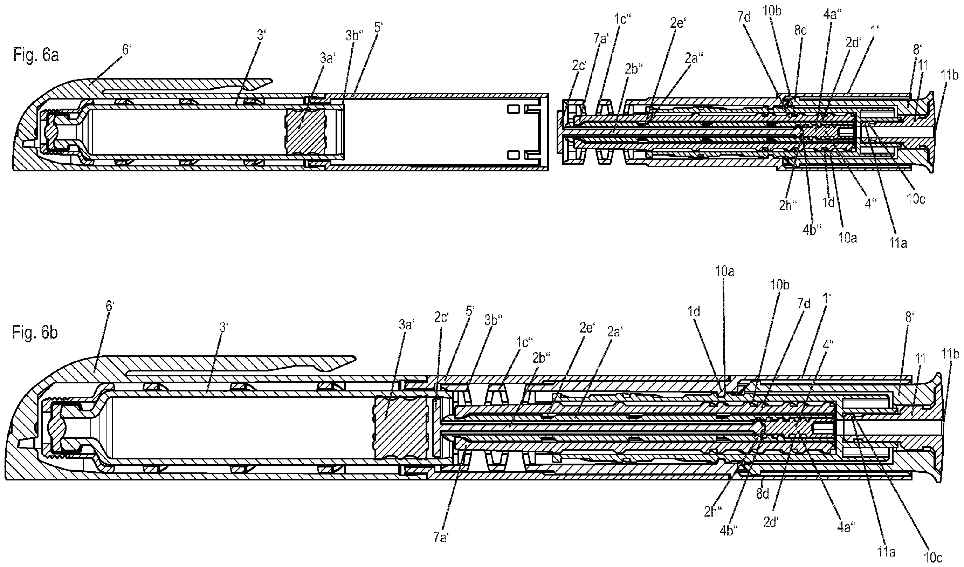

Die dritte Ausführungsform der erfindungsgemässen Injektionsvorrichtung mit einer Längsachse (L) umfasst ein Gehäuse (1') mit einem distalen und einem proximalen Ende. Das Gehäuse (1') weist ein Halteelement (1c") in Form einer Haltefeder auf, um einen in einem Behälterhalter (5') aufgenommenen Behälter (3') spielfrei relativ zu dem Behälterhalter (5') in die distale Richtung zu bewegen oder zu drücken. Ferner umfasst die dritte Ausführungsform der erfindungsgemässen Injektionsvorrichtung eine Rotationshülse (10), eine Vorschubhülse (7') und einen Dosisknopf (8'). Das Gehäuse (1') weist ein Innengewinde (1d) auf, welches mit einem Aussengewinde (10a) der Rotationshülse (10) in einem ersten Gewindeeingriff (1d; 10a) ist. Zudem umfasst die Rotationshülse (10), ein Innengewinde (Figur 6a; 10b), welches in einem zweiten Gewindeeingriff (Figur 6a, 10b; 7d) mit einem an der Vorschubhülse (7') vorgesehenen Aussengewinde (7d) ist. Der erste (1d; 10a) und der zweite Gewindeeingriff (Figur 6a, 10b; 7d) weisen vorzugsweise die gleiche Steigungsrichtung auf. Der erste Gewindeeingriff (1d; 10a) weist zudem vorzugweise eine grössere Gewindesteigung als der zweite Gewindeeingriff (Figur 6a, 10b; 7d) auf. Der zweite Gewindeeingriff (Figur 6a, 10b; 7d) weist vorzugweise eine geringere Gewindesteigung als der erste Gewindeeingriff (1d; 10a) auf. Dadurch kann eine Übersetzung, insbesondere eine Weg- und/oder eine Kraftübersetzung eines Ladevorgangs und/oder eines Ausschüttvorgangs einer festgelegten Dosis erzielt werden. Ferner umfasst die Injektionsvorrichtung eine mindestens zweiteilige Kolbenstange (2a"; 2b"), um einen in dem Behälter (3') aufgenommenen Stopfen (Figur 6a, 3a') in die distale Richtung zu bewegen, sodass das fluide Produkt ausgeschüttet wird. Die mindestens zweiteilige Kolbenstange (2a"; 2b") umfasst ein Kolbenstangenmittel (2a") und einen Kolbenstangenfuss (2b"). Der Kolbenstangenfuss (2b") ist zumindest teilweise von dem Kolbenstangenmittel (2a") aufgenommen, wobei der Kolbenstangenfuss (2b) und das Kolbenstangenmittel (2a) relativ zueinander axial bewegbar und relativ zueinander drehfest angeordnet sind. Die Injektionsvorrichtung umfasst ferner einen Einsatz (4"), welcher an einem proximalen Ende des Kolbenstangenfusses (2b") vorgesehen ist. Der Einsatz (4") weist ein Aussengewinde (4a") auf, welches in einem Gewindeeingriff (4a"; Figur 6a, 2d") mit einem Innengewinde (Figur 6a, 2d') an dem Kolbenstangenmittel (2a") ist. Bei relativer Drehung zwischen dem Einsatz (4") und dem Kolbenstangenmittel (2a") ist der Kolbenstangenfuss (2b") relativ zu dem Kolbenstangenmittel (2a") axial bewegbar, sodass das distale Ende des Kolbenstangenfusses (2b"), insbesondere ein axialfest angeordneter Flansch (2c') des Kolbenstangenfusses (2b") und der Stopfen (Figur 6a, 3a') aneinander liegen oder in einen axialen Anschlagkontakt kommen. Der axiale Abstand zwischen dem Kolbenstangenfuss (2b"), insbesondere zwischen dem Flansch (2c') des Kolbenstangenfusses (2b") und dem Stopfen (Figur 6a, 3a') wird während dem Zusammenbauen der Injektionsvorrichtung bestimmt. Des Weiteren kann das proximale Ende des Kolbenstangenfusses (2b") vorzugsweise eine Verzahnung (Figur 6a, 2h") umfassen und das distale Ende des Einsatzes (4") kann vorzugsweise eine Verzahnung (4b") aufweisen, wobei die beiden Verzahnungen (Figur 6a, 2h"; 4b") relativ zueinander in eine erste Drehrichtung um die Längsachse (L) drehbar angeordnet sind. Die beiden Verzahnungen (Figur 6a, 2h"; 4b") können vorzugsweise derart ausgebildet sein, dass eine relative Drehung in eine zweite der ersten Drehrichtung entgegengesetzten Drehrichtung blockiert ist.

Die dritte Ausführungsform der erfindungsgemässen Injektionsvorrichtung weist ferner eine Dosiskappe (11) auf, welche an dem proximalen Ende des Dosisknopfs (8') angeordnet ist. Die Dosiskappe (11) ist vorzugweise axialfest, insbesondere axial- und drehfest mit dem Dosisknopf (8') verbunden. Alternativ kann die Dosiskappe (11) und der Dosisknopf (8') einteilig ausgebildet sein. Der Dosisknopf (8') ist an dem proximalen Ende des Gehäuses (1') angeordnet und kann relativ zu dem Gehäuse (1') axial hin und her bewegt werden, um eine festgelegte Dosis des fluiden Produkts aufzuziehen und aus dem Behälter (3') auszuschütten. Um die Injektionsvorrichtung zu verwenden, zieht der Benutzer den Dosisknopf (8') in die proximale Richtung, um eine festgelegte Dosis zu laden. Der Dosisknopf (8') ist relativ zu dem Gehäuse (1') axial bewegbar und drehfest angeordnet. Die Rotationshülse (10) ist relativ zu dem Gehäuse (1') drehbar und axial bewegbar angeordnet. Die Dosiskappe (11) umfasst eine Haltenocke (11a), welche in einem Eingriff mit einer an der Rotationshülse (10) vorgesehenen Ringnut (Figur 6a, 10c) ist, um eine axialfeste und drehbare Verbindung zwischen der Rotationshülse (10) und der Dosiskappe (11) und/oder des Dosisknopfes (8') zu bilden. Alternativ kann zwischen dem Dosisknopf (8') und der Rotationshülse (10) eine axialfeste und drehbare Verbindung vorgesehen sein, um eine axialfeste und drehbare Verbindung zwischen der Rotationshülse (10) und der Dosiskappe (11) und/oder des Dosisknopfes (8') zu bilden. Beim Laden der festgelegten Dosis wird aufgrund der axialfesten Verbindung zwischen der Rotationshülse (10) und der Dosiskappe (11) und/oder dem Dosisknopf die Rotationshülse (10) zusammen mit dem Dosisknopf (8') um die gleiche Distanz in die proximale Richtung bewegt. Aufgrund des ersten Gewindeeingriffs (10a; 1d) zwischen der Rotationshülse (10) und des Gehäuses (1') wird die Rotationshülse (10) beim Laden der festgelegten Dosis helixförmig in die proximale Richtung bewegt, wobei die Vorschubhülse (7') aufgrund des zweiten Gewindeeingriffs (7d; Figur 6a, 10b) zwischen der Vorschubhülse (7') und der Rotationshülse (10') und der relativ zu dem Gehäuse (1') drehfesten Anordnung um eine geringe Distanz als die Rotationshülse (10) oder des Dosisknopfs (8') in die proximale Richtung verschoben wird. Die Vorschubhülse (7') weist einen Halteschnapper (7a') auf, welche mit einer an dem Kolbenstangenmittel (2a") vorgesehenen Zahnung (2e') zusammenwirken kann. Der unter Kraftaufwand lösbare Halteschnapper (7a') der Vorschubhülse (7') wirkt derart mit der Zahnung (2e') des Kolbenstangenmittels (2a") zusammen, dass bei einer axialen Bewegung des Dosisknopfs (8') relativ zu dem Gehäuse (1') in die proximale Richtung der Halteschnapper (7a') der Vorschubhülse (7') über die Zahnung (2e') des Kolbenstangenmittels (2a") in die proximale Richtung gleitet. Das Kolbenstangenmittel (2a") wird dabei relativ zu dem Gehäuse (1') axialfest gehalten. Dazu weist das Gehäuse (1') einen Halteschnapper (nicht ersichtlich) auf, welcher derart mit der Zahnung (2e') des Kolbenstangenmittels (2a") zusammenwirkt, dass bei der axialen Bewegung des Dosisknopfs (8') relativ zu dem Gehäuse (1') in die proximale Richtung der Halteschnapper (nicht ersichtlich) des Gehäuses (1') das Kolbenstangenmittel (2a") relativ zu dem Gehäuse (1') axialfest hält. Beim Ausschütten der festgelegten Dosis drückt der Benutzer den Dosisknopf (8') in die distale Richtung. Aufgrund der axialfesten Verbindung zwischen der Rotationshülse (10) und der Dosiskappe (11) und/oder des Dosisknopfs (8') wird die Rotationshülse (10) und der Dosisknopf (8') um die gleiche Distanz in die distale Richtung bewegt, wobei die Vorschubhülse (7') aufgrund des zweiten Gewindeeingriffs (7d; Figur 6a, 10b) zwischen der Vorschubhülse (7') und der Rotationshülse (10') und der relativ zu dem Gehäuse (1') drehfesten Anordnung um eine geringe Distanz als die Rotationshülse (10) oder des Dosisknopfs (8') in die distale Richtung verschoben wird. Der Halteschnapper (7a') der Vorschubhülse (7') wirkt derart mit der Zahnung (2e') des Kolbenstangenmittels (2a") zusammen, dass bei der axialen Bewegung des Dosisknopfs (8') relativ zu dem Gehäuse (1') in die distale Richtung der Halteschnapper (7a') der Vorschubhülse (7') das Kolbenstangenmittel (2a") mit Hilfe der Zahnung (2e') des Kolbenstangemittels (2a") in einem Eingriff hält, sodass die Vorschubhülse (7') das Kolbenstangenmittel (2a") in die distale Richtung mitnimmt. Dabei wird das Kolbenstangenmittel (2a") in distale Richtung bewegt. Aufgrund der Verrastung der beiden Verzahungen zwischen dem Einsatz (4") und dem Kolbenstangenfuss (2b') sind das Kolbenstangenmittel (2a") und der Kolbenstangenfuss (2b") relativ zueinander axialfest, insbesondere axial- und drehfest verbunden. Das Kolbenstagenmittel (2a") bewegt somit über den Kolbenstangenfuss (2b"), insbesondere über den mit dem Kolbenstangenfuss (2b") verbundenen Flansch (2c') den Stopfen (3a') in die distale Richtung, um das fluide Produkt auszuschütten. Dabei wirkt der unter Kraftaufwand lösbare Halteschnapper (nicht ersichtlich) des Gehäuses (1') derart mit der Zahnung (2e') des Kolbenstangenmittels (2a") zusammen, dass bei einer axialen Bewegung des Dosisknopfs (8') relativ zu dem Gehäuse (1') in die distale Richtung der Halteschnapper (nicht ersichtlich) des Gehäuses (1') über die Zahnung (2e) des Kolbenstangenmittels (2a") gleitet und in die nachfolgende Zahnung (2e') des Kolbenstangenmittels (2a"), welche proximal zu der vorangehenden Zahnung (2e') an dem Kolbenstangenmittel (2a) angeordnet ist, eingreift. Dabei wird das Kolbenstangenmittel (2a") in die distale Richtung bewegt und schüttet die festgelegte Dosis aus. Die festgelegte Dosis wird durch die entsprechenden axialen Anschlagkontakte und/oder Drehanschlagkontakte zwischen einem distalen und einem proximalen nach innen ragenden Vorsprung (nicht ersichtlich) an dem Gehäuse (1') und einer nach aussen ragenden Nocke (8d) an dem Dosisknopf (8') definiert. Um eine erneute festgelegt Dosis zuladen, zieht der Benutzer den Dosisknopf (8') erneut in die proximale Richtung. Am Ende der letzten ausgeschütteten Dosis ist ein an der Vorschubhülse (7') vorgesehener Anschlag (nicht ersichtlich) in einem axialen Anschlagkontakt und/oder in einem Drehanschlagkontakt mit einer an dem proximalen Ende oder in einem proximalen Bereich des Kolbenstangenmittels (2a) vorgesehenen Anschlagschulter (2f'). Eine weitere festgelegte Dosis kann somit nicht geladen werden.

In der Figur 6a ist eine Längsschnittansicht eines ersten Montageschritts der dritten Ausführungsform der erfindungsgemässen Injektionsvorrichtung gemäss Figur 5 dargestellt.

Die Montageschritte der ersten und der dritten Ausführungsform der erfindungsgemässen Injektionsvorrichtung unterscheiden sich zueinander durch einzelne Montageschritte, welche durch die Funktion des Ladens und des Ausschüttens einer festgelegten Dosis resultieren.

In einem Montageschritt wird eine Behälterhaltereinheit gebildet. Die Behälterhaltereinheit wird gemäss den Montageschritten der ersten Ausführungsform der erfindungsgemässen Injektionsvorrichtung zusammengebaut und umfasst wie diese Behälterhaltereinheit eine Verschlusskappe (6') und den mit dem Stopfen (3a') aufgenommenen Behälter (3'), wobei der Behälter (3') in den Behälterhalter (5') eingesetzt ist.

In einem weiteren Montageschritt wird eine Antriebseinheit gebildet. Zur Bildung der Antriebseinheit wird zuerst das Kolbenstangenmittel (2a") in die Vorschubhülse (7') gesetzt. Das Kolbenstangenmittel (2b") wird dabei durch das proximale Ende der Vorschubhülse (7') geschoben und relativ zu der Vorschubhülse (7') in die distale Richtung verschoben. In einem weiteren Montageschritt wird der Einsatz (4") über die Gewindeverbindung (2d'; 4a") zwischen dem Innengewinde (2d') des Kolbenstangenmittels (2a") und dem Aussengewinde (4a") des Einsatzes (4") in das Kolbenstangenmittel (2a") eingeschraubt. Der Einsatz (4") wird dabei durch das proximale Ende des Kolbenstangenmittels (2a") in die distale Richtung in das Kolbenstangenmittel (2b") eingeschraubt. In einem weiteren Montageschritt wird die Rotationshülse (10) auf das proximale Ende der mit dem Kolbenstangenmittel (2a") aufgenommenen Vorschubhülse (7') aufgesetzt und mit Hilfe der zweiten Gewindeverbindung (7d; 10b) zwischen der Vorschubhülse (7') und der Rotationshülse (10) relativ zu der Vorschubhülse (7') in die distale Richtung geschraubt. In einem weiteren Montageschritt wird der Dosisknopf (8) auf das proximale Ende der mit dem Kolbenstangenmittel (2a) aufgenommenen Vorschubhülse (7) aufgesetzt und relativ zu der Vorschubhülse (7) in die distale Richtung verschoben. In einem weiteren Montageschritt wird die Baueinheit bestehend aus der Rotationshülse (10), der Vorschubhülse (7'), dem Einsatz (4") und des Kolbenstangenmittels (2a") in das Gehäuse (1') gesetzt. Diese Baueinheit wird dabei auf das proximale Ende des Gehäuses (1') gesetzt und mit Hilfe der ersten Gewindeverbindung (1d; 10a) zwischen dem Gehäuse (1') und der Rotationshülse (10) relativ zu dem Gehäuse (1') in die distale Richtung geschraubt. In einem weiteren Montageschritt wird die Dosiskappe (11) mit dem Dosisknopf (8') axialfest, insbesondere axial- und drehfest verbunden. Die Baueinheit bestehend aus der Dosiskappe (11) und dem Dosisknopf (8') wird in einem weiteren Montageschritt in das Gehäuse (1') gesetzt. Diese Baueinheit wird dabei auf das proximale Ende des Gehäuses (1') gesetzt. Diese Baueinheit wird dabei durch das proximale Ende des Gehäuses (1') axial relativ zu dem Gehäuse (1') in die distale Richtung verschoben, um eine axialfeste Verbindung zwischen der Dosiskappe (11) und der Rotationshülse (10) zu bilden. In einem weiteren Montageschritt wird der Kolbenstangenfuss (2b") in das Kolbenstangenmittel (2a") gesetzt. Der Kolbenstangenfuss (2b") wird dabei durch das distale Ende des Kolbenstangenmittels (2b) geschoben und relativ zu dem Kolbenstangenmittel (2b) in die proximale Richtung verschoben, wobei der Flange (2c') an dem distalen Ende des Kolbenstangenfusses (2b") vorgesehen ist.

In einem weiteren Montageschritt werden die Behälterhaltereinheit mit der Antriebseinheit zusammengefügt, wie in der Figur 6b ersichtlich ist. In diesem Montageschritt wird der mit dem Behälter (3') aufgenommenen Behälterhalter (5') mit dem den Kolbenstangenfuss (2b"), das Kolbenstangenmittel (2a") und den Einsatz (4") aufgenommenen Gehäuse (1') axialfest verbunden. Es können eine Schnappverbindung, eine plastische Verformung, eine Schraubverbindung, eine Klebeverbindung oder eine Schweissverbindung vorgesehen sein. Das an dem Gehäuse (1') vorgesehene Halteelement (1c"), insbesondere die Haltefeder gelangt beim Zusammenfügen der Behälterhaltereinheit mit der Antriebseinheit in einen Anschlagkontakt mit einem proximalen Rand (3b") des Behälters (3'), um den Behälter (3') relativ zu dem Behälterhalter (5') spielfrei in die distale Richtung zu drücken.

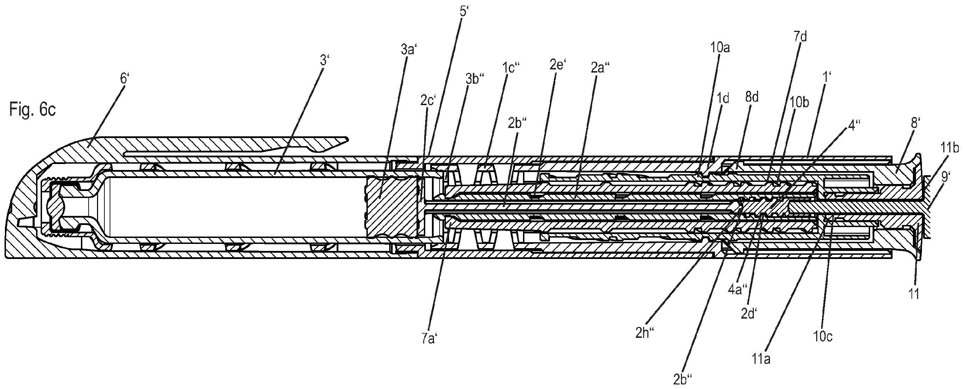

In einem weiteren Montageschritt, wie in der Figur 6c gezeigt ist, wird der axiale Abstand zwischen dem Kolbenstangenfuss (2b"), insbesondere der axiale Abstand zwischen dem mit dem Kolbenstangenfuss (2b") axialfest verbundenen Flange (2c') und dem Stopfen (3a') der Injektionsvorrichtung derart bestimmt, dass ein distales Ende des Kolbenstangenfusses (2b"), insbesondere der dem mit dem Kolbenstangenfuss (2b") axialfest verbundenen Flange (2c') und der Stopfen (3a') aneinander zu liegen kommt.

Ein Werkzeug (9') wird durch eine Öffnung (11b) an dem proximalen Ende der Dosiskappe (11) in den Einsatz (4") geschoben. Der Einsatz (4) weist insbesondere eine Öffnung an dem proximalen Ende auf. Das Werkzeug (9') gelangt in einen Eingriff, insbesondere in einen drehfesten Eingriff mit dem Einsatz (4"). Das Werkzeug (9') dreht den Einsatz (4") relativ zu dem Kolbenstangenmittel (2a") aufgrund der Gewindeverbindung (4a"; 2d') zwischen dem Einsatz (4") und dem Kolbenstangenmittel (2a") in die erste Drehrichtung um die Längsachse (L). Die Drehung des Werkzeugs (9') in die erste Drehrichtung um die Längsachse (L) wird auf den Einsatz (4") übertragen.

Durch diese Drehung des Einsatzes (4") oder des mit dem Werkzeug (9') verbundenen Einsatzes (4") kann der axiale Abstand zwischen dem Kolbenstangenfuss (2b"), insbesondere zwischen dem mit dem Kolbenstangenfuss (2b") axialfest verbundenen Flange (2c') und dem Stopfen (3a') derart bestimmt werden, dass das distale Ende des Kolbenstangenfusses (2b"), insbesondere der mit dem Kolbenstangenfuss (2b") axialfest verbundene Flange (2c') und der Stopfen (3a') aneinander zu liegen kommen. Der Kolbenstangenfuss (2b") wird innerhalb des Kolbenstangenmittels (2a") durch die kombinierte Axial- und Drehbewegung des Einsatzes (4") und einem Anschlagkontakt zwischen dem Einsatz (4") und dem Kolbenstangenfuss (2b") relativ zu dem Kolbenstangenmittel (2a") in die distale Richtung bewegt oder verschoben. Während der relativen Drehung zwischen dem Einsatz (4") und dem Kolbenstangenmittel (2a") in die erste Drehrichtung um die Längsachse (L) nimmt die Drehkraft zu, um beim Erreichen eines bestimmten Schwellenwerts anzuzeigen, dass das distale Ende des Kolbenstangenfusses (2b"), insbesondere der mit dem Kolbenstangenfuss (2b") axialfest verbundene Flange (2c') in einem axialen Anschlagkontakt mit dem Stopfen (3a') ist. Ein Drehmomentanstieg, insbesondere ein sprunghafter Drehmomentanstieg, welcher detektiert werden kann, zeigt an, dass das distale Ende des Kolbenstangenfusses (2b"), insbesondere der mit dem Kolbenstangenfuss (2b") axialfest verbundene Flange (2c') in einem axialen Anschlagkontakt mit dem Stopfen (3a') ist. Somit kann genau festgestellt werden, wann das distale Ende des Kolbenstangenfusses (2b"), insbesondere der mit dem Kolbenstangenfuss (2b") axialfest verbundene Flange (2c') und der Stopfen (3a') in einem axialen Anschlagkontakt sind. Bei diesem Schwellenwert ist die Verzahnung (4b"; 2h") zwischen dem Einsatz (4") und dem Kolbenstangenfuss (2b") verrastet. Diese Verrastung zwischen dem Einsatz (4") und dem Kolbenstangenfuss (2b") bewirkt, dass das Kolbenstangenmittel (2a") und der Kolbenstangenfuss (2b"), insbesondere über den Einsatz (4") relativ zueinander axialfest verbunden sind. Somit ist die mindestens zweiteilige Kolbenstange (2a"; 2b") gebildet, welche den in dem Behälter (3') aufgenommenen Stopfen (3a') in die distale Richtung bewegen kann, um das fluide Produkt auszuschütten.

In einem weiteren Montageschritt kann die Öffnung (11b) an dem proximalen Ende der Dosiskappe (11) beispielsweise mit einer Etikette oder einer weiteren Kappe verschlossen werden.

In einer weiteren Ausführungsform der erfindungsgemässen Injektionsvorrichtung kann die Injektionsvorrichtung gemäss der dritten Ausführungsform einen Einsatz gemäss der zweiten Ausführungsform aufweisen. Die Funktion dieser weiteren Ausführungsform der erfindungsgemässen Injektionsvorrichtung, nämlich das Laden und das Ausschütten einer festgelegten Dosis entspricht somit der Funktion der dritten Ausführungsform der erfindungsgemässen Injektionsvorrichtung. Die Anordnung und Ausgestaltung des Einsatzes gemäss der weiteren Ausführungsform der erfindungsgemässen Injektionsvorrichtung entspricht aber der Anordnung und Ausgestaltung des Einsatzes gemäss der zweiten Ausführungsform der erfindungsgemässen Injektionsvorrichtung.

Bezugszeichen:
- 1; 1': Gehäuse
- 1a: Fenster des Gehäuses
- 1b: Verbindungselement des Gehäuses
- 1c; 1c'; 1c": Halteelement
- 1d: Innengewinde des Gehäuses
- 2a; 2a'; 2a": Kolbenstangenmittel
- 2b; 2b'; 2b": Kolbenstangenfuss
- 2c; 2c': Flansch
- 2d; 2d': Innengewindes des Kolbenstangenmittels
- 2e; 2e': Zahnung des Kolbenstangenmittels
- 2f; 2f': Anschlagschulter
- 2g: Innengewinde des Kolbenstangenfusses
- 2h; 2h": Verzahnung des Kolbenstangenfusses
- 2h': Verzahnung des Kolbenstangenmittels
- 3; 3': Behälter
- 3a; 3a': Stopfen
- 3b; 3b'; 3b": proximaler Rand des Behälters
- 4; 4'; 4": Einsatz
- 4a; 4a';4a": Aussengewinde des Einsatzes
- 4b; 4b'; 4b": Verzahnung des Einsatzes
- 5; 5': Behälterhalter
- 5a: Fenster des Behälterhalters
- 5b: Aussengewinde des Behälterhalters
- 5c: Verbindungselement des Behälterhalters
- 6; 6': Verschlusskappe
- 7; 7': Vorschubhülse
- 7a; 7a': Halteschnapper
- 7b: Nocke der Vorschubhülse
- 7c: Anschlag
- 7d: Aussengewinde der Vorschubhülse
- 8: Dosisknopf
- 8a: Aussparung
- 8b: Markierung
- 8c: Öffnung des Dosisknopfs
- 8d: Nocke des Dosisknopfs
- 9; 9': Werkzeug
- 10: Rotationshülse
- 10a: Aussengewinde der Rotationshülse
- 10b: Innengewinde der Rotationshülse
- 10c: Ringnut
- 11: Dosiskappe
- 11a: Haltenocke
- 11b: Öffnung der Dosiskappe

## Patentansprüche

1. Injektionsvorrichtung zur Verabreichung eines fluiden Produkts mit einer Längsachse (L) umfassend:
- ein Gehäuse (1; 1') mit einem distalen und einem proximalen Ende,
- einen Behälter (3; 3') mit dem fluiden Produkt und einen entlang der Längsachse (L) axial bewegbaren Stopfen (3a; 3a'),
- eine mindestens zweiteilige Kolbenstange (2a; 2a'; 2a"), welche zumindest teilweise oder vollständig in dem Gehäuse (1; 1') vorgesehen ist und welche den in dem Behälter (3; 3') aufgenommenen Stopfen (3a; 3a') in die distale Richtung bewegen kann, um das fluide Produkt auszuschütten,
- wobei die mindestens zweiteilige Kolbenstange (2a; 2a'; 2a"; 2b; 2b'; 2b") ein Kolbenstangenmittel (2a; 2a'; 2a") und einen Kolbenstangenfuss (2b; 2b'; 2b") umfasst und der Kolbenstangenfuss (2b; 2b'; 2b") zumindest teilweise von dem Kolbenstangenmittel (2a; 2a'; 2a") aufgenommen ist,
- wobei ferner der Kolbenstangenfuss (2b; 2b'; 2b") und das Kolbenstangemittel (2b; 2b'; 2b") relativ zueinander axial bewegbar und relativ zueinander drehfest angeordnet sind und der axiale Abstand zwischen dem Kolbenstangenfuss (2b; 2b'; 2b") und dem Stopfen (3a; 3a') während dem Zusammenbauen der Injektionsvorrichtung bestimmt wird und derart bestimmt wird, dass ein distales Ende des Kolbenstangenfusses (2b; 2b'; 2b") und der Stopfen (3a; 3a') aneinander liegen,
**dadurch gekennzeichnet, dass**
- ein Einsatz (4; 4'; 4") an einem proximalen Ende des Kolbenstangenfusses (2b; 2b'; 2b") und/oder innerhalb des Kolbenstangenfusses (2b; 2b'; 2b") vorgesehen ist, wobei der Einsatz (4; 4'; 4") ein Aussengewinde (4a; 4a'; 4a") aufweist, welches in einem Gewindeeingriff (4a; 4a'; 4a"; 2h; 2h'; 2g) mit einem Innengewinde (2h; 2h') an dem Kolbenstangenmittel (2a; 2a'; 2a") oder mit einem Innengewinde (2g) an dem Kolbenstangenfuss (2b; 2b'; 2b") ist und,
- wobei bei relativer Drehung zwischen dem Einsatz (4; 4'; 4") und dem Kolbenstangenmittel (4a; 4a'; 4a") oder dem Kolbenstangenfuss (2b; 2b'; 2b"), der Kolbenstangenfuss (2b; 2b'; 2b") relativ zu dem Kolbenstangenmittel (2a; 2a'; 2a") axial bewegbar ist, sodass das distale Ende des Kolbenstangenfusses (2b; 2b'; 2b") und der Stopfen (3a; 3a') aneinander liegen.

2. Injektionsvorrichtung nach Anspruch 1, **dadurch gekennzeichnet, dass** an dem proximalen Ende des Kolbenstangenfusses (2b; 2b") eine Verzahnung (2h; 2h") und an einem distalen Ende des Einsatzes (4; 4") eine Verzahnung (4b; 4b") vorgesehen ist, wobei die beiden Verzahnungen (2h; 2h"; 4b; 4b") relativ zueinander in eine erste Drehrichtung um die Längsachse (L) drehbar angeordnet sind.

3. Injektionsvorrichtung nach Anspruch 1, **dadurch gekennzeichnet, dass** an dem proximalen Ende des Kolbenstangenmittels (4a') eine Verzahnung (2h') und an einem proximalen Ende des Einsatzes (4') eine Verzahnung (4b') vorgesehen ist, wobei die beiden Verzahnungen (2h'; 4b') relativ zueinander in eine erste Drehrichtung um die Längsachse (L) drehbar angeordnet sind.

4. Injektionsvorrichtung nach Anspruch 2 oder 3, **dadurch gekennzeichnet, dass** die beiden Verzahnungen (2h; 2h' 2h"; 4b; 4b'; 4b") derart ausgebildet sind, dass eine relative Drehung in eine zweite der ersten Drehrichtung entgegengesetzten Drehrichtung blockiert ist.

5. Verfahren zum Zusammenbauen einer Vorrichtung nach einem der Ansprüche 1 bis 4 mit den folgenden Schritten:
- relative Drehung des Einsatzes (4; 4'; 4") zu dem Kolbenstangenmittel (2a; 2a'; 2a") oder zu dem Kolbenstangenfuss (2b; 2b'; 2b") in eine erste Drehrichtung
- relative axiale Bewegung des Kolbenstangenfusses (2b; 2b'; 2b") zu dem Kolbenstangenmittel (2a; 2a'; 2a")
- axialer Anschlag zwischen dem distalen Ende des Kolbenstangenfusses (2b; 2b'; 2b") und des Stopfens (3a; 3a'), wobei das distale Ende des Kolbenstangenfusses (2b; 2b'; 2b") und der Stopfen (3a; 3a') aneinander zu liegen kommen.

6. Verfahren nach Anspruch 5, **dadurch gekennzeichnet, dass** während der relativen Drehung zwischen dem Einsatz (4; 4'; 4") und dem Kolbenstangenmittel (2a; 2a'; 2a") oder dem Kolbenstangenfuss (2b; 2b'; 2b") in die erste Drehrichtung die Drehkraft zunimmt, um beim Erreichen eines Schwellenwerts anzuzeigen, dass das distale Ende des Kolbenstangenfusses (2b; 2b'; 2b") in axialen Anschlagkontakt mit dem Stopfen (3a; 3a') ist.

7. Verfahren nach Anspruch 5 oder 6, **dadurch gekennzeichnet, dass** der Einsatz (4; 4'; 4") relativ zu dem Kolbenstangenmittel (2a; 2a'; 2a") oder zu dem Kolbenstangenfuss (2b; 2b'; 2b") in die erste Drehrichtung gedreht werden kann und dass eine relative Drehung zwischen dem Einsatz (4; 4'; 4") und dem Kolbenstangenmittel (2a; 2a'; 2a") oder dem Kolbenstangenfuss (2b; 2b'; 2b") in eine zweite der ersten Drehrichtung entgegengesetzten Drehrichtung blockiert wird.

8. Verfahren nach einem der Ansprüche 5 bis 7, **dadurch gekennzeichnet, dass** der Einsatz (4; 4'; 4") mit einem Werkzeug (9; 9') in einen Eingriff, insbesondere in einen drehfesten Eingriff gelangen kann und das Werkzeug (9; 9') den Einsatz (4; 4'; 4") relativ zu dem Kolbenstangenmittel (2a; 2a'; 2a") oder dem Kolbenstangenfuss (2b; 2b'; 2b") in die erste Drehrichtung drehen kann.

9. Verfahren nach einem der Ansprüche 5 bis 8, **dadurch gekennzeichnet, dass** der axiale Abstand zwischen dem Kolbenstangenfuss (2b; 2b'; 2b") und dem Stopfen (3a; 3a') derart bestimmt wird, dass ein distales Ende des Kolbenstangenfusses (2b; 2b'; 2b") und der Stopfen (3a; 3a') aneinander zu liegen kommt.

10. Verfahren nach einem der Ansprüche 5 bis 9, **dadurch gekennzeichnet, dass** in einem Schritt ein mit dem Behälter (3; 3') aufgenommenen Behälterhalter (5; 5') mit dem den Kolbenstangenfuss (2b; 2b'; 2b"), das Kolbenstangenmittel (2a; 2a'; 2a") und den Einsatz (4; 4'; 4") aufgenommenen Gehäuse (1; 1') axialfest verbunden, insbesondere verschnappt, plastisch verformt, verschraubt, verklebt oder verschweisst wird und in einem weiteren Schritt der axiale Abstand zwischen dem Kolbenstangenfuss (2b; 2b'; 2b") und dem Stopfen (3a; 3a') der Injektionsvorrichtung derart bestimmt wird, dass ein distales Ende des Kolbenstangenfusses (2b; 2b'; 2b") und der Stopfen (3a; 3a') aneinander zu liegen kommt.

11. Verfahren nach Anspruch 10, **dadurch gekennzeichnet, dass** der Einsatz (4; 4") mit einem Aussengewinde (4a; 4a") in das Kolbenstangenmittel (2a; 2a") mit einem Innengewinde (2d; 2d') geschraubt wird und der Kolbenstangenfuss (2b; 2b") durch das distale Ende des Kolbenstangenmittels (2a; 2a") eingesetzt wird.

12. Verfahren nach Anspruch 10, **dadurch gekennzeichnet, dass** der Einsatz (4') mit einem Aussengewinde (4a') in den Kolbenstangenfuss (2b') mit einem Innengewinde (2g) geschraubt wird und der mit dem Einsatz (4') aufgenommenen Kolbstangenfuss (4b) in das Kolbenstangemittel (4a) eingesetzt wird.
